(19) **Europäisches Patentamt**
**European Patent Office**
**Office européen des brevets**

(11) **EP 4 492 048 A1**

(12) **EUROPÄISCHE PATENTANMELDUNG**

(43) Veröffentlichungstag:
**15.01.2025   Patentblatt 2025/03**

(21) Anmeldenummer: **23185034.8**

(22) Anmeldetag: **12.07.2023**

(51) Internationale Patentklassifikation (IPC):
**G01N 27/04** *(2006.01)*     **G01N 33/208** *(2019.01)*

(52) Gemeinsame Patentklassifikation (CPC):
**G01N 27/041; G01N 33/208**

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**KH MA MD TN**

(71) Anmelder: **Heraeus Precious Metals GmbH & Co. KG**
**63450 Hanau (DE)**

(72) Erfinder:
• **WEGNER, Matthias**
  **63450 Hanau (DE)**
• **SCHUSTER, Jochen**
  **63450 Hanau (DE)**
• **KROHN, Luca**
  **63450 Hanau (DE)**

(74) Vertreter: **Heraeus IP**
**Heraeus Business Solutions GmbH**
**Intellectual Property**
**Heraeusstraße 12-14**
**63450 Hanau (DE)**

(54) **VERFAHREN ZUR MESSUNG EINER ELEKTRISCHEN GRÖSSE ZUR BESTIMMUNG DER ZEITDAUER EINER DISPERSIONSHÄRTUNG EINES METALLHALTIGEN FORMKÖRPERS**

(57)     Die vorliegende Erfindung betrifft die Verwendung einer Messanordnung zur Bestimmung der Zeitdauer einer thermischen Dispersionshärtung eines Metallblechs (1), das eine Vorderseite (2), eine Rückseite, Seitenflächen (3), Längskanten (4) und Querkanten (5) aufweist,
wobei das Metallblech (1) über eine erste elektrische Kontaktfläche (11a) und eine zweite elektrische Kontaktfläche mit einer Strom- oder Spannungsquelle (12) und über eine dritte elektrische Kontaktfläche (13a) und eine vierte elektrische Kontaktfläche (13b) mit einem Spannungs- oder Strommessgerät (14) verbunden ist, wobei die erste elektrische Kontaktfläche (11a) vollständig innerhalb einer Fläche liegt, die aus den Schnittflächen (8a, 8b) der Vorderseite (2), Rückseite und Seitenflächen (3) mit einem gedachten ersten Zylinder besteht, wobei die Zylinderachse des gedachten ersten Zylinders durch eine der Ecken der Vorderseite (2) läuft,
die zweite elektrische Kontaktfläche vollständig innerhalb einer Fläche liegt, die aus den Schnittflächen (10a, 10b) der Vorderseite (2), Rückseite und Seitenflächen (3) mit einem gedachten zweiten Zylinder besteht, wobei die Zylinderachse des gedachten zweiten Zylinders durch die Ecke der Vorderseite (2) läuft, die zu der Ecke, durch die die Zylinderachse des gedachten ersten Zylinders läuft, den größten Abstand aufweist.

Figur 6

**Beschreibung**

Es ist allgemein bekannt, dass sich die mechanische Festigkeit metallischer Werkstoffe durch Dispersionshärtung deutlich erhöhen lässt, insbesondere für Anwendungen bei hoher Temperatur. Dispersionsgehärtete Metalle enthalten nichtmetallische Partikel, beispielsweise Oxidpartikel, die in einer Metallmatrix dispergiert sind. Erfolgt die Dispersionshärtung durch Oxidpartikel, so werden die damit erhaltenen Metalle auch als "ODS"-Metalle (ODS: *"Oxide Dispersion Strengthened"*) bezeichnet.

Es kann in diesem Zusammenhang beispielhaft auf folgende Lehrbuchauszüge verwiesen werden:

- G. Gottstein, "Materialwissenschaft und Werkstofftechnik-Physikalische Grundlagen", 4. Auflage, 2014, SpringerVieweg, Seite 276 ("Dispersionshärtung")
- A.C. Reardon (Editor), "Metallurgy for the Non-Metallurgist", 2. Auflage, 2011, ASM International, S. 69-70 ("Other Important Strengthening Mechanisms - Dispersion Strengthening")
- W.D. Callister and D.G. Rethwisch, "Materials Science and Engineering - An Introduction", 10. Auflage, 2018, Wiley, Seite 571 ("Dispersion-Strengthened Composites")

Da die in der Metallmatrix dispergierten anorganischen Partikel auch bei hohen Temperaturen thermisch stabil sind, sich also beispielsweise auch bei hohen Temperaturen nicht in der umgebenden Metallmatrix lösen, sind dispersionsgehärtete Metalle insbesondere für Hochtemperaturanwendungen interessant.

Eine Dispersionshärtung kann beispielsweise pulvermetallurgisch durch mechanisches Mahlen (z.B. Einmahlen oxidischer Partikel in eine metallische Matrix) mit anschließender Kompaktierung durch einen Sinterprozess erfolgen.

Alternativ kann in einem schmelzmetallurgischen Verfahren zunächst eine Ausgangslegierung hergestellt werden, die in geringer Konzentration oxidierbare Legierungselemente enthält. Durch Behandlung der Ausgangslegierung in einer oxidativen Atmosphäre werden die oxidierbaren Legierungselemente in Oxidpartikel überführt und es wird ein dispersionsverfestigter Werkstoff, der eine Metallmatrix und darin dispergierte Oxidpartikel enthält, erhalten. Diese Art der Dispersionshärtung wird auch als "innere Oxidation" bezeichnet.

Die Dispersionshärtung kann mit einer Vielzahl unterschiedlicher Metalle durchgeführt werden.

Formkörper aus Platin werden vielfach in Hochtemperaturprozessen eingesetzt, bei denen das Material eine hohe Korrosionsbeständigkeit aufweisen muss. Beispielsweise werden platinhaltige Bauteile wie Rührer oder Glasfaserdüsenwannen in der Glasindustrie verwendet. Da Platin bei hohen Temperaturen eine geringe mechanische Festigkeit aufweist, werden für Anwendungen in Hochtemperaturprozessen häufig Bauteile aus dispersionsgehärteten Platinlegierungen eingesetzt.

Die Herstellung, Bearbeitung und die physikalischen Eigenschaften derartiger dispersionsgehärteter Platin-Zusammensetzungen sind beispielsweise aus den Druckschriften EP 3 971 311 B1, GB 1 280 815 A, GB 1 340 076 A, GB 2 082 205 A, EP 0 683 240 A2, EP 0 870 844 A1, EP 0 947 595 A2, EP 1 188 844 A1, EP 1 295 953 A1, EP 1 964 938 A1, US 2 636 819 A, US 4 507 156 A, DE 23 55 122 A1, WO 81/01013 A1 und WO 2015/082630 A1 bekannt.

Bei der Herstellung eines dispersionsverfestigten Formkörpers wird beispielsweise zunächst ein Halbzeug (z.B. ein Blech oder ein Rohr) unter Verwendung einer Ausgangslegierung schmelzmetallurgisch (z.B. durch Gießen in eine Form) hergestellt, gegebenenfalls durch Umformungsprozesse in die gewünschte Form überführt und anschließend einer thermischen Behandlung in einer oxidativen Atmosphäre unterzogen, so dass sich in der Metallmatrix dispergierte Oxidpartikel ausbilden können.

Die Herstellung von dispersionsverfestigten Legierungen ist ein komplexer und zeitaufwendiger Prozess. Die Ausbildung der Oxidpartikel in der Metallmatrix durch innere Oxidation erfolgt durch Eindiffundieren von Sauerstoff in den Formkörper.

Die Legierungselemente, die der Legierung zur Ausbildung der Oxidpartikel zugegeben wurden, sollten im gesamten Volumen der Legierung möglichst vollständig oxidiert werden. Je größer das Volumen des zu härtenden Materials, umso länger ist üblicherweise die erforderliche Zeitdauer, um eine vollständige Oxidation zu realisieren. Dies trifft insbesondere auf Halbzeuge wie Bleche oder Rohre zu, die im Vergleich zu Drähten in mindestens zwei Raumrichtungen eine signifikante Ausdehnung haben.

Der Oxidationsgrad einer Legierung lässt sich beispielsweise durch Bestimmung des Sauerstoffgehalts über quantitative IR-Spektroskopie bestimmen. Diese Methode erfordert jedoch, dass dem zu untersuchenden Material zunächst eine definierte Probenmenge entnommen wird und diese entnommene Probe anschließend unter Verwendung eines NDIR-Sensors spektroskopisch analysiert wird. Alternativ kann der Oxidationsgrad der Probe durch Flächenanalyse im metallographischen Schliff untersucht werden, was ebenfalls eine Probenpräparation unter Beschädigung des Formkörpers erfordert. Außerdem muss in beiden Fällen (d.h. der Messung des Sauerstoffgehaltes mittels IR-Spektroskopie oder Flächenanalyse im metallographischen Schliff) der innere Oxidationsprozess unterbrochen werden und erst nach erfolgter Messung kann entschieden werden, ob das Material vollständig oxidiert ist oder der Oxidationsprozess fortgesetzt werden muss.

M. Bruncko et al., "In-situ monitoring of internal oxidation ofdilute alloys", Corrosion Science, 49, 2007, S. 1228-1244, beschreiben eine Methode, bei der während der Dispersionshärtung einer Legierung deren elektrischer Widerstand über eine Vierleitermessung bestimmt wurde, um Rückschlüsse auf den Oxidationsgrad der Legierung und die erforderliche Zeitdauer der Dispersionshärtung zu ziehen. Die Messung erfolgte an einem Draht (Länge: 150 mm; Durchmesser: 0,5 mm) und somit einem im Wesentlichen eindimensionalen Formkörper.

Eine Aufgabe der vorliegenden Erfindung ist die Bereitstellung einer Messmethode, die während der Dispersionshärtung eines Formkörpers, der in mindestens zwei Raumrichtungen eine signifikante Ausdehnung aufweist, insbesondere eines Blechs, Rohrs oder Stabes, verlässliche Rückschlüsse auf den Oxidationsgrad des Formkörpers ermöglicht, ohne dass die Dispersionshärtung unterbrochen oder der Formkörper beschädigt werden muss.

Gemäß einer ersten Ausführungsform der Erfindung wird die Aufgabe gelöst durch ein Verfahren zur Messung einer elektrischen Größe eines metallhaltigen Formkörpers während einer thermischen Dispersionshärtung des metallhaltigen Formkörpers, wobei

der metallhaltige Formkörper ein Metallblech ist, das eine Vorderseite, eine der Vorderseite gegenüberliegende Rückseite und Seitenflächen, die die Vorder- und Rückseite miteinander verbinden, aufweist, wobei

die Vorderseite und die Rückseite jeweils Längskanten, deren Länge $L_{Längskante}$ mindestens 50 mm beträgt, Querkanten, deren Länge $L_{Querkante}$ mindestens 50 mm beträgt, und Ecken, in denen sich die Längs- und Querkanten treffen, aufweisen,

das Metallblech eine erste elektrische Kontaktfläche und eine zweite elektrische Kontaktfläche aufweist, die mit einer Strom- und/oder Spannungsquelle verbunden werden, wobei

die erste elektrische Kontaktfläche vollständig innerhalb einer Fläche liegt, die aus den Schnittflächen der Vorderseite, der Rückseite und der Seitenflächen mit einem gedachten ersten Zylinder besteht, wobei die Zylinderachse des gedachten ersten Zylinders senkrecht auf der Vorderseite des Metallblechs steht und durch eine der Ecken der Vorderseite läuft, der Zylindermantel des gedachten ersten Zylinders die Längskante der Vorderseite in einem Abstand a1 von der Zylinderachse und die Querkante der Vorderseite in einem Abstand b1 von der Zylinderachse schneidet, wobei

$$a1 \leq 0,2 \times L_{Längskante} \text{ und } b1 \leq 0,2 \times L_{Querkante}$$

wobei

$L_{Längskante}$ die Länge der Längskante und $L_{Querkante}$ die Länge der Querkante sind, die sich in der Ecke treffen, durch die die Zylinderachse des gedachten ersten Zylinders läuft,

die zweite elektrische Kontaktfläche vollständig innerhalb einer Fläche liegt, die aus den Schnittflächen der Vorderseite, der Rückseite und den Seitenflächen mit einem gedachten zweiten Zylinder besteht, wobei die Zylinderachse des gedachten zweiten Zylinders senkrecht auf der Vorderseite des Metallblechs steht und durch die Ecke der Vorderseite läuft, die zu der Ecke, durch die die Zylinderachse des gedachten ersten Zylinders läuft, den größten Abstand aufweist, der Zylindermantel des gedachten zweiten Zylinders die Längskante der Vorderseite in einem Abstand a2 von der Zylinderachse und die Querkante der Vorderseite in einem Abstand b2 von der Zylinderachse schneidet, wobei

$$a2 \leq 0,2 \times L_{Längskante} \text{ und } b2 \leq 0,2 \times L_{Querkante}$$

wobei

$L_{Längskante}$ die Länge der Längskante und $L_{Querkante}$ die Länge der Querkante sind, die sich in der Ecke treffen, durch die die Zylinderachse des gedachten zweiten Zylinders läuft,

das Metallblech eine dritte elektrische Kontaktfläche und eine vierte elektrische Kontaktfläche aufweist, die mit einem Spannungsmessgerät verbunden werden, sofern die erste und zweite elektrische Kontaktfläche mit einer Stromquelle verbunden werden, oder die mit einem Strommessgerät verbunden werden, sofern die erste und zweite elektrische Kontaktfläche mit einer Spannungsquelle verbunden werden,

das Metallblech, das über seine erste und zweite elektrische Kontaktfläche mit der Strom- oder Spannungsquelle und über seine dritte und vierte elektrische Kontaktfläche mit dem Spannungs- oder Strommessgerät verbunden ist, thermisch behandelt wird, so dass sich in dem Metallblech Oxidpartikel ausbilden und

während der thermischen Behandlung der elektrische Widerstand oder eine zum elektrischen Widerstand proportionale elektrische Größe des Metallblechs bestimmt wird.

Bei dem erfindungsgemäßen Verfahren handelt es sich um eine Vierleitermessung. Mit dieser kann, wie dem Fachmann prinzipiell bekannt ist, der elektrische (Ohmsche) Widerstand oder eine dazu proportionale elektrische Messgröße eines Prüfkörpers bestimmt werden. Wie jedoch nachfolgend eingehender beschrieben wird, ist es im Rahmen der vorliegenden Erfindung wesentlich, wo die Verbindungen zu der Stromquelle (oder alternativ der Spannungsquelle) auf der Oberfläche des Metallblechs angebracht werden.

Wie jedes Metallblech weist das Metallblech, an dem das erfindungsgemäße Messverfahren durchgeführt wird, eine Vorderseite, eine der Vorderseite gegenüberliegende Rückseite und Seitenflächen, die die Vorder- und Rückseite miteinander verbinden, auf. Die Vorderseite und die Rückseite weisen jeweils Längskanten, Querkanten und Ecken, in denen sich die Längs- und Querkanten treffen, auf. Die Längs- und Querkanten weisen Längen von mindestens 50 mm auf.

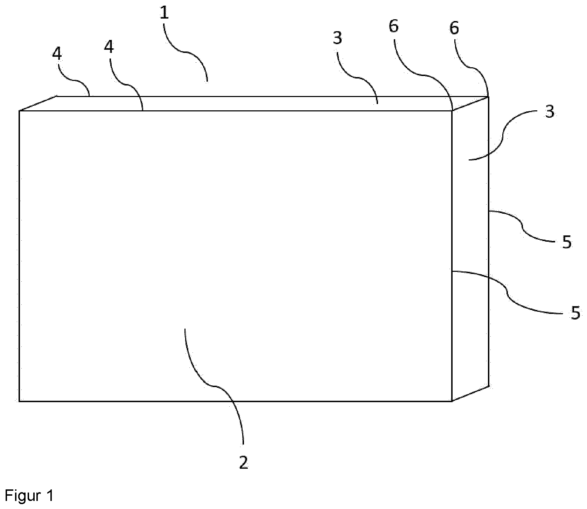

Ein solches beispielhaftes Metallblech, an dem das erfindungsgemäße Messverfahren durchgeführt werden kann, wird in Figur 1 veranschaulicht.

Das in Figur 1 veranschaulichte Metallblech 1 weist eine Vorderseite 2 auf. Gegenüber der Vorderseite 2 befindet sich die Rückseite des Metallblechs. Vorderseite 2 und Rückseite des Blechs sind über die Seitenflächen 3 miteinander verbunden und weisen jeweils Längskanten 4, Querkanten 5 und Ecken 6, in denen sich die Längskanten 4 und Querkanten 5 treffen, auf.

In einer beispielhaften Ausführungsform weisen die Längskanten eine Länge $L_{\text{Längskante}}$ von 50 mm bis 2000 mm und/oder die Querkanten eine Länge $L_{\text{Querkante}}$ von 50 mm bis 2000 mm auf. Die Vorder- und Rückseite des Metallblechs können beispielsweise eine im Wesentlichen quadratische Form aufweisen (d.h. $L_{\text{Längskante}}$ und $L_{\text{Querkante}}$ sind gleich oder weisen zumindest sehr ähnliche Werte auf) oder alternativ eine längliche Form aufweisen (d.h. $L_{\text{Längskante}}$ und $L_{\text{Querkante}}$ weisen deutlich unterschiedliche Werte auf). Die Längskanten und Querkanten der Vorderseite oder Rückseite stehen beispielsweise im Wesentlichen senkrecht (z.B. in einem Winkel von 90° +/- 10°) zueinander. Das Metallblech weist beispielsweise eine Dicke von maximal 15 mm auf.

Das Metallblech ist somit ein Formkörper, der in mindestens zwei Raumrichtungen eine signifikante Ausdehnung aufweist. Dementsprechend gibt es prinzipiell viele Möglichkeiten, wie das Metallblech im Rahmen einer Vierleitermessung zur Bestimmung seines elektrischen Widerstands mit der Stromquelle (oder alternativ der Spannungsquelle) kontaktiert werden kann.

In dem Verfahren der vorliegenden Erfindung wird die Vierleitermessung während der Dispersionshärtung des Metallblechs durchgeführt. Im Rahmen der vorliegenden Erfindung wurde erkannt, dass sich aus der Messung des elektrischen Widerstands (oder einer zum elektrischen Widerstand proportionalen elektrischen Größe wie z.B. der elektrischen Spannung oder Stromstärke) eines Metallblechs über eine Vierleitermessung verlässlichere Rückschlüsse auf den Oxidationsgrad und damit den Fortschritt der Dispersionshärtung ziehen lassen, wenn die Kontaktierung des Metallblechs mit der Stromquelle (oder alternativ der Spannungsquelle) so erfolgt, dass sich die erste elektrische Kontaktfläche relativ nah an einer ersten Ecke des Metallblechs befindet, während sich die zweite elektrische Kontaktfläche möglichst nah an der Ecke, die gegenüber der ersten Ecke den größten Abstand aufweist, befindet. Bei einem viereckigen Blech sind dies die diagonal gegenüberliegenden Ecken.

Die erste elektrische Kontaktfläche liegt vollständig innerhalb einer Fläche, die sich zusammensetzt aus den Schnittflächen der Vorderseite, der Rückseite und den Seitenflächen des Metallblechs mit einem gedachten ersten Zylinder. Die Schnittflächen der Vorderseite, Rückseite und Seitenflächen des Metallblechs mit dem gedachten ersten Zylinder sind die Flächen der Vorderseite, Rückseite und Seitenflächen, die innerhalb des gedachten ersten Zylinders liegen. Wie jeder Zylinder weist dieser gedachte erste Zylinder eine Zylinderachse und einen Zylindermantel auf. Die Zylinderachse des gedachten ersten Zylinders steht senkrecht auf der Vorderseite des Metallblechs und läuft durch eine der Ecken, in der sich eine der Längskanten und eine der Querkanten der Vorderseite treffen. Der Zylindermantel des gedachten ersten Zylinders schneidet die Längskante der Vorderseite in einem Abstand a1 von der Zylinderachse und die Querkante der Vorderseite in einem Abstand b1 von der Zylinderachse, wobei

$$a1 \leq 0{,}2 \times L_{\text{Längskante}} \text{ und } b1 \leq 0{,}2 \times L_{\text{Querkante}}$$

wobei

$L_{\text{Längskante}}$ die Länge der Längskante und $L_{\text{Querkante}}$ die Länge der Querkante sind, die sich in der Ecke treffen, durch den die Zylinderachse des gedachten ersten Zylinders läuft Der gedachte erste Zylinder kann ein Kreiszylinder oder ein elliptischer Zylinder sein. Der Abstand eines Punktes von der Zylinderachse ist die Länge der kürzesten Verbindungslinie zwischen diesem Punkt und der Zylinderachse.

In einer beispielhaften Ausführungsform genügen a1 und $L_{\text{Längskante}}$ sowie b1 und $L_{\text{Querkante}}$ den folgenden Beziehungen:

$$a1 \leq 0{,}14 \times L_{\text{Längskante}} \text{ und } b1 \leq 0{,}14 \times L_{\text{Querkante}}$$

Die zweite elektrische Kontaktfläche liegt vollständig innerhalb einer Fläche, die aus den Schnittflächen der Vorderseite, der Rückseite und den Seitenflächen des Metallblechs mit einem gedachten zweiten Zylinder besteht. Die Schnittflächen der Vorderseite, Rückseite und Seitenflächen des Metallblechs mit dem gedachten zweiten Zylinder sind die Flächen der Vorderseite, Rückseite und Seitenflächen, die innerhalb des gedachten zweiten Zylinders liegen. Die Zylinderachse des gedachten zweiten Zylinders steht senkrecht auf der Vorderseite des Metallblechs und läuft durch die Ecke der Vorderseite, die zu der Ecke, durch die die Zylinderachse des gedachten ersten Zylinders läuft, den größten Abstand aufweist. Der Zylindermantel des gedachten zweiten Zylinders schneidet die Längskante der Vorderseite in einem Abstand a2 von der Zylinderachse und die Querkante der Vorderseite in einem Abstand b2 von der Zylinderachse, wobei

$$a2 \leq 0{,}2 \times L_{\text{Längskante}} \text{ und } b2 \leq 0{,}2 \times L_{\text{Querkante}}$$

wobei

$L_{\text{Längskante}}$ die Länge der Längskante und $L_{\text{Querkante}}$ die Länge der Querkante sind, die sich in der Ecke treffen, durch die die Zylinderachse des gedachten zweiten Zylinders läuft. Der gedachte zweite Zylinder kann ein Kreiszylinder oder ein elliptischer Zylinder sein. Der Abstand eines Punktes von der Zylinderachse ist die Länge der kürzesten Verbindungslinie zwischen diesem Punkt und der Zylinderachse.

In einer beispielhaften Ausführungsform genügen a2 und $L_{\text{Längskante}}$ sowie b2 und $L_{\text{Querkante}}$ den folgenden Beziehungen:

$$a2 \leq 0{,}14 \times L_{\text{Längskante}} \text{ und } b2 \leq 0{,}14 \times L_{\text{Querkante}}$$

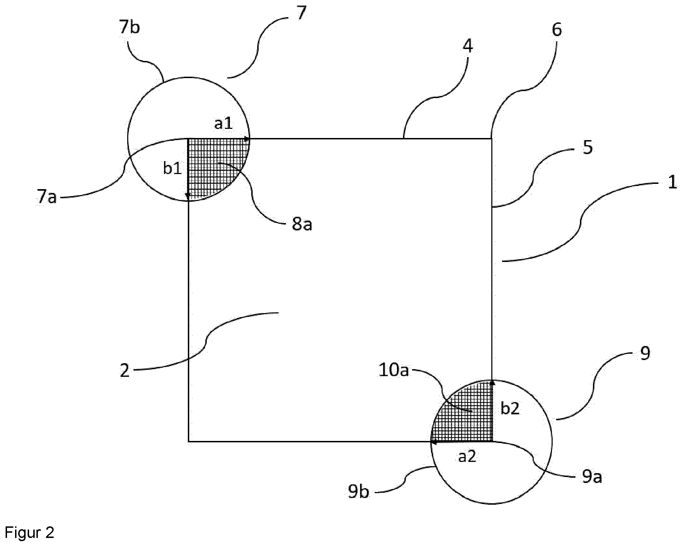

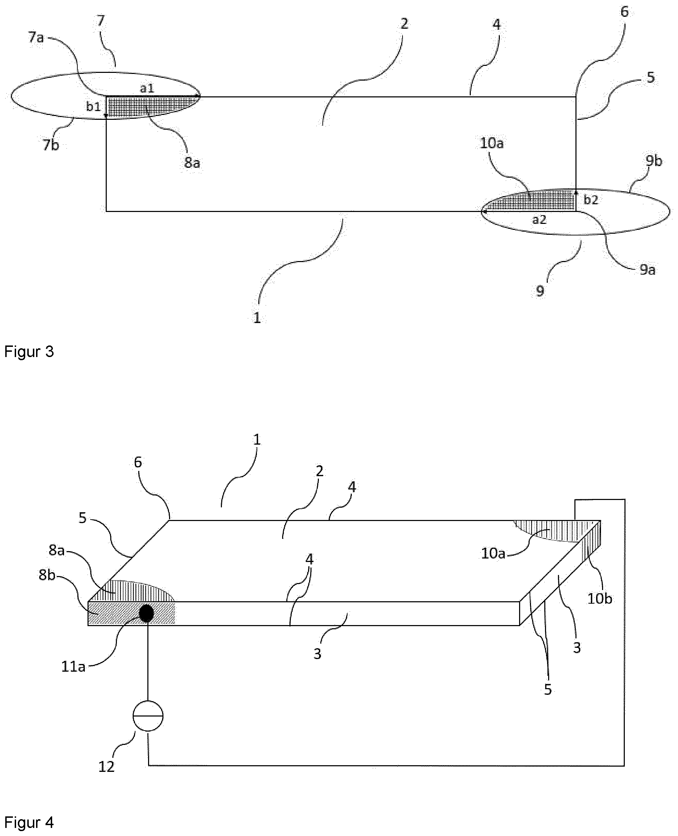

Die gedachten ersten und zweiten Zylinder und die Schnittflächen der Vorderseite und Seitenflächen des Metallblechs mit diesen gedachten Zylindern sind in Figur 2 ($L_{\text{Längskante}} = L_{\text{Querkante}}$) und Figur 3 ($L_{\text{Längskante}} \gg L_{\text{Querkante}}$) veranschaulicht.

Figur 2 zeigt eine Draufsicht der Vorderseite 2 des Metallblechs 1, wobei Längskante 4 und Querkante 5 der Vorderseite 2 eine gleiche Länge aufweisen und sich in den Ecken 6 treffen. Die Zylinderachse 7a des gedachten ersten Zylinders 7 läuft duch eine der Ecken 6 und steht senkrecht auf der Vorderseite 2 des Metallblechs 1. Der Zylindermantel 7b des gedachten ersten Zylinders schneidet die Längskante 4 in einem Abstand a1 von der Zylinderachse 7a und die Querkante 5 in einem Abstand b1 von der Zylinderachse 7a. Da die Vorderseite 2 eine quadratische Form aufweist (d.h. $L_{\text{Längskante}} = L_{\text{Querkante}}$), gilt: a1 = b1 = 0,2 x $L_{\text{Längskante}}$ = 0, 2 x $L_{\text{Querkante}}$. Die Vorderseite 2 schneidet den gedachten ersten Zylinder 7 unter Erhalt einer Schnittfläche 8a (schraffierte Fläche in Figur 2). Neben dieser Schnittfläche 8a gibt es noch weitere Schnittflächen (nicht gezeigt in Figur 2) der Seitenflächen und der Rückseite des Metallblechs mit dem gedachten ersten Zylinder. Die erste elektrische Kontaktfläche, über die eine Verbindung des Metallblechs 1 zur Strom- oder Spannungsquelle der Messanordnung erfolgt, liegt innerhalb der Fläche, die sich aus der Schnittfläche 8a der Vorderseite 2, der Schnittfläche der Rückseite und der Schnittflächen der Seitenflächen mit dem gedachten ersten Zylinder 7 zusammensetzt (d.h. aus diesen Schnittflächen besteht). Die Zylinderachse 9a des gedachten zweiten Zylinders 9 steht senkrecht auf der Vorderseite 2 des Metallblechs 1 und läuft durch die Ecke 6 der Vorderseite 2, die zu der Ecke 6, durch die die Zylinderachse 7a des gedachten ersten Zylinders 7 läuft, den größten Abstand aufweist. Der Zylindermantel 9b des gedachten zweiten Zylinders schneidet die Längskante 4 in einem Abstand a2 von der Zylinderachse 9a und die Längskante 5 in einem Abstand b2 von der Zylinderachse 9a. Auch für den gedachten zweiten Zylinder gilt: a2 = b2 = 0,2 x $L_{\text{Längskante}}$ = 0, 2 x $L_{\text{Querkante}}$. Die Schnittfläche 10a der Vorderseite 2 mit dem gedachten zweiten Zylinder ist schraffiert. Neben dieser Schnittfläche gibt es noch weitere Schnittflächen (nicht gezeigt in Figur 2) der Seitenflächen und der Rückseite des Metallblechs mit dem gedachten zweiten Zylinder 9. Die zweite elektrische Kontaktfläche, über die eine Verbindung des Metallblechs zur Strom- oder Spannungsquelle der Messanordnung erfolgt, ist innerhalb dieser Schnittflächen zu wählen.

Figur 3 zeigt eine Draufsicht der Vorderseite 2 des Metallblechs 1, wobei Längskante 4 und Querkante 5 der Vorderseite 2 deutlich unterschiedliche Längen aufweisen und sich in den Ecken 6 treffen. Der Zylindermantel 7b des gedachten ersten Zylinders 7 schneidet die Längskante 4 in einem Abstand a1 von der Zylinderachse 7a und die Längskante 5 in einem Abstand b1 von der Zylinderachse 7a, wobei a1 = 0,2 x $L_{\text{Längskante}}$ und b1 = 0,2 x $L_{\text{Querkante}}$. Die Vorderseite 2 des Metallblechs 1 schneidet den gedachten ersten Zylinder 7 in der Schnittfläche 8a. Der Zylindermantel 9b des gedachten zweiten Zylinders 9 schneidet die Längskante 4 in einem Abstand a2 von der Zylinderachse 9a und die Längskante 5 in einem Abstand b2 von der Zylinderachse 9a, wobei a2 = 0,2 x $L_{\text{Längskante}}$ und b2 = 0,2 x $L_{\text{Querkante}}$. Die

Vorderseite 2 des Metallblechs 1 schneidet den gedachten zweiten Zylinder in der Schnittfläche 10a.

Wie oben bereits angemerkt, sind die elektrischen Kontaktflächen, mit denen das Metallblech mit der Strom- oder Spannungsquelle verbunden wird, so zu wählen, dass sie jeweils innerhalb einer Fläche liegen, die aus den Schnittflächen der Vorderseite, Rückseite und Seitenflächen des Metallblechs mit dem gedachten ersten oder zweiten Zylinder bestehen.

In der in Figur 4 veranschaulichten beispielhaften Ausführungsform schneidet die Vorderseite 2 des Metallblechs 1 den gedachten ersten Zylinder (nicht gezeigt in Figur 4) in der Schnittfläche 8a und den gedachten zweiten Zylinder (nicht gezeigt in Figur 4) in der Schnittfläche 10a. Die Seitenflächen 3 schneiden den ersten und zweiten gedachten Zylinder unter anderem in den Schnittflächen 8b und 10b. Die erste elektrische Kontaktfläche 11a für die Verbindung mit der Stromquelle 12 liegt auf der Schnittfläche 8b, während die zweite elektrische Kontaktfläche (nicht gezeigt in Figur 4) auf einer Schnittfläche der gegenüberliegenden Seitenfläche mit dem gedachten zweiten Zylinder liegt.

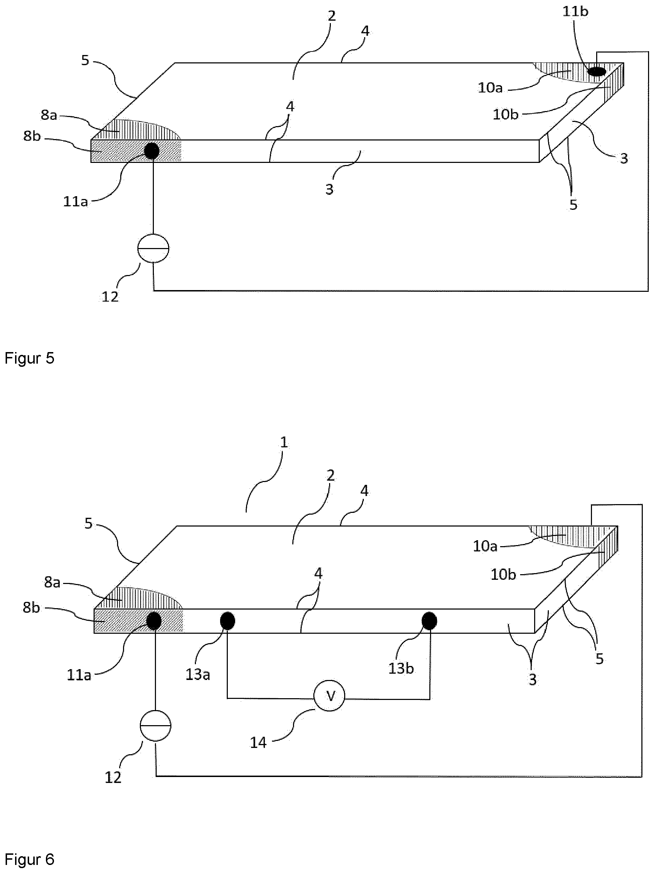

In Figur 5 entsprechen die Schnittflächen 8a, 8b, 10a und 10b der Vorderseite 2 und der Seitenflächen 3 mit den gedachten Zylindern den in Figur 4 gezeigten Schnittflächen. Ebenfalls in Übereinstimmung mit Figur 4 befindet sich die erste elektrische Kontaktfläche 11a für die Verbindung mit der Stromquelle 12 innerhalb der Schnittfläche 8b. Die zweite elektrische Kontaktfläche 11b befindet sich in der in Figur 5 veranschaulichten beispielhaften Ausführungsform innerhalb der Schnittfläche 10a.

Über eine dritte elektrische Kontaktfläche und eine vierte elektrische Kontaktfläche des Metallblechs wird eine Verbindung des Metallblechs mit einem Spannungs- oder Strommessgerät hergestellt. Sofern die erste und zweite elektrische Kontaktfläche des Metallblechs mit einer Stromquelle verbunden werden, erfolgt eine Verbindung der dritten und vierten elektrischen Kontaktfläche des Metallblechs mit einem Spannungsmessgerät. Sofern die erste und zweite elektrische Kontaktfläche des Metallblechs mit einer Spannungsquelle verbunden werden, erfolgt eine Verbindung der dritten und vierten elektrischen Kontaktfläche des Metallblechs mit einem Strommessgerät.

Für die Kontaktierung der elektrischen Kontaktflächen des Metallblechs mit der Strom- oder Spannungsquelle und dem Spannungs- oder Strommessgerät können Kontaktelemente verwendet werden, die dem Fachmann bekannt sind. Beispielsweise erfolgt das Verbinden der elektrischen Kontaktflächen mit der Strom- oder Spannungsquelle und dem Spannungs- oder Strommessgerät über Klemmen (z.B. Kelvinklemmen oder Krokodilklemmen) oder Kontaktstifte.

Thermische Dispersionshärtungen erfolgen üblicherweise bei recht hohen Temperaturen und manchmal über recht lange Zeiträume. Um unter diesen Bedingungen auf einfache und effiziente Weise einen verlässlichen elektrischen Kontakt zwischen dem Metallblech und der Strom- oder Spannungsquelle zu realisieren, kann es bevorzugt sein, die erste oder zweite elektrische Kontaktfläche mit einer elektrischen Leitung der Strom- oder Spannungsquelle und die dritte und vierte elektrische Kontaktfläche mit den elektrischen Leitungen des Spannungs- oder Strommessgeräts unter Einwirkung der Schwerkraft des Metallblechs zu kontaktieren, beispielsweise indem das Metallblech (beispielsweise über seine Vorderseite oder eine seiner Seitenflächen) auf die elektrischen Leitungen gelegt oder gestellt wird. Das Metallblech wird beispielsweise mit einer seiner Seitenflächen auf die elektrischen Leitungen gestellt. In dieser bevorzugten Ausführungsform kann die Anzahl der erforderlichen Kontaktklemmen deutlich reduziert werden.

Eine geeignete Größe der elektrischen Kontaktflächen auf dem Metallblech kann der Fachmann auf Basis seines Fachwissens oder gegebenenfalls durch Routineversuche bestimmen. Beispielsweise weisen die elektrischen Kontaktflächen des Metallblechs jeweils eine Fläche von mindestens 1 mm$^2$ auf.

In dem erfindungsgemäßen Verfahren ist wesentlich, dass die erste elektrische Kontaktfläche und die zweite elektrische Kontaktfläche des Metallblechs (d.h. die Kontaktflächen des Metallblechs, über die die Verbindung des Metallblechs mit der Strom- oder Spannungsquelle erfolgt) so gewählt sind, dass sie jeweils innerhalb einer Fläche liegen, die aus den Schnittflächen der Vorderseite, Rückseite und Seitenflächen mit den gedachten Zylindern besteht. Dies stellt sicher, dass sich die erste elektrische Kontaktfläche relativ nahe an einer der Ecken des Metallblechs befindet, während sich die zweite elektrische Kontaktfläche möglichst nah an der gegenüberliegenden Ecke befindet.

Die Positionen der dritten und vierten elektrischen Kontaktfläche auf dem Metallblech (d.h. die Kontaktflächen des Metallblechs, über die die Verbindung des Metallblechs mit dem Spannungs- oder Strommessgerät erfolgt) können frei gewählt werden. Geeignete Positionen kann der Fachmann auf Basis seines allgemeinen Fachwissens und gegebenenfalls durch Routineversuche ermitteln.

Beispielsweise weisen die dritte und vierte elektrische Kontaktfläche

von der Ecke, durch die die Zylinderachse des gedachten ersten Zylinders läuft, einen größeren Abstand auf als die erste elektrische Kontaktfläche und

von der Ecke, durch die die Zylinderachse des gedachten zweiten Zylinders läuft, einen größeren Abstand auf als die zweite elektrische Kontaktfläche. Der Abstand ist die kürzeste gerade Verbindungslinie zwischen der Kontaktfläche und der Ecke.

Beispielsweise liegen die dritte und vierte elektrische Kontaktfläche außerhalb der Schnittflächen der Vorderseite, Rückseite und Seitenflächen mit dem gedachten ersten Zylinder und außerhalb der Schnittflächen der Vorderseite, Rückseite und Seitenflächen mit dem gedachten zweiten Zylinder.

Beispielsweise weisen die dritte und vierte elektrische Kontaktfläche einen Abstand voneinander auf, der geringer ist als der Abstand zwischen der ersten und zweiten elektrischen Kontaktfläche. Der Abstand ist die kürzeste gerade Verbindungslinie zwischen den beiden jeweiligen Kontaktflächen.

Beispielsweise liegen die erste, dritte und vierte elektrische Kontaktfläche auf derselben Seitenfläche des Metallblechs vor und die zweite elektrische Kontaktfläche liegt auf einer anderen Seitenfläche, bevorzugt der gegenüberliegenden Seitenfläche des Metallblechs vor. Diese beispielhafte Ausführungsform ist in Figur 6 veranschaulicht. Hinsichtlich der Schnittflächen 8a, 8b, 10a und 10b der Vorderseite 2 und der Seitenflächen 3 mit den gedachten Zylindern und der Positionen der ersten elektrischen Kontaktfläche 11a und der zweiten elektrischen Kontaktfläche (für die Verbindung mit der Stromquelle 12) entspricht Figur 6 der in Figur 4 veranschaulichten Ausführungsform. Über die elektrischen Kontaktflächen 13a und 13b ist das Metallblech 1 mit dem Spannungsmessgerät 14 verbunden.

Das Metallblech, das über die erste und zweite elektrische Kontaktfläche mit der Strom- oder Spannungsquelle und über die dritte und vierte elektrische Kontaktfläche mit dem Spannungs- oder Strommessgerät verbunden ist, wird thermisch behandelt, so dass sich in dem Metallblech Oxidpartikel ausbilden.

Geeignete Metalle in Form von Legierungen, die bei einer thermischen Behandlung in einer oxidierenden Atmosphäre Oxidpartikel ausbilden (so dass eine Metallmatrix und darin dispergierte Oxidpartikel erhalten werden) und für eine Dispersionshärtung geeignet sind, sind dem Fachmann bekannt.

Beispielsweise ist das Metall, aus dem das Metallblech gefertigt ist, eine Edelmetalllegierung, die ein oder mehrere Nicht-Edelmetalle enthält. Beispielsweise enthält die Edelmetalllegierung Nicht-Edelmetalle in einer Konzentration von nicht mehr als 1 Gew% und der Rest sind ein oder mehrere Edelmetalle und unvermeidliche Verunreinigungen. Das Nicht-Edelmetall ist beispielsweise ausgewählt aus Zirkonium, Cer, Scandium und Yttrium und das Edelmetall ist beispielsweise ausgewählt aus Ruthenium, Rhodium, Gold, Palladium, Platin, Iridium und Osmium. Die Edelmetalllegierung enthält beispielsweise ein erstes Edelmetall als Hauptelement und weitere Edelmetalle, sofern vorhanden, in einer Gesamtkonzentration von maximal 29,95 Gew%, unter der Maßgabe, dass sich das erste Edelmetall von den weiteren Edelmetallen unterscheidet. "Hauptelement" bedeutet, dass dieses Element gegenüber jedem der anderen Elemente in einer höheren Konzentration vorliegt.

Geeignete Temperaturen für die Durchführung der Dispersionshärtung sind dem Fachmann bekannt oder gegebenenfalls durch Routineversuche bestimmbar. Beispielsweise erfolgt die thermische Behandlung des Metallblechs bei einer Temperatur von mindestens 750°C, z.B. im Bereich von 750°C bis 1400°C oder 800°C bis 1200°C.

Die Dispersionshärtung erfolgt in einer oxidierenden Atmosphäre. Geeignete oxidierende Atmosphären für Dispersionshärtungen von Legierungen sind dem Fachmann bekannt. Beispielsweise ist die oxidierende Atmosphäre eine sauerstoffhaltige Atmosphäre. Beispielsweise beträgt der Sauerstoffgehalt der oxidierenden Atmosphäre mindestens 5 Vol%, bevorzugter mindestens 10 Vol%. In einer beispielhaften Ausführungsform wird Luft als oxidierende Atmosphäre verwendet.

Während der thermischen Behandlung wird der elektrische Widerstand oder eine zum elektrischen Widerstand proportionale elektrische Größe des Metallblechs bestimmt.

Der elektrische Widerstand (auch als Ohmscher Widerstand bezeichnet) wird mit der bekannten Beziehung $R = U/I$ bestimmt, wobei

I die Stromstärke des mit der Stromquelle (verbunden mit der ersten und zweiten elektrischen Kontaktfläche des Metallblechs) eingespeisten Stroms und U die mit dem Spannungsmessgerät (verbunden mit der dritten und vierten elektrischen Kontaktfläche des Metallblechs) gemessene Spannung sind oder

U die mit der Spannungsquelle (verbunden mit der ersten und zweiten elektrischen Kontaktfläche des Metallblechs) angelegte Spannung und I die mit dem Strommessgerät (verbunden mit der dritten und vierten elektrischen Kontaktfläche des Metallblechs) gemessene Stromstärke sind.

Der gemessene elektrische Widerstand wird durch den Oxidationsgrad des Metallblechs beeinflusst. Die thermische Behandlung wird beispielsweise beendet, wenn der elektrische Widerstand des Metallblechs über eine festgelegte Zeitdauer (z.B. mindestens 1 Stunde oder mindestens 4 Stunden) einen konstanten Wert (z.B. eine Schwankung von maximal +/- 1%) zeigt.

Anstelle des elektrischen Widerstands des Metallblechs kann eine zu dem elektrischen Widerstand proportionale Größe, z.B. die elektrische Spannung oder Stromstärke, herangezogen werden, um die erforderliche Zeitdauer für die Dispersionshärtung zu bestimmen. Beispielsweise kann es ausreichend sein, die Spannung oder die Stromstärke an der dritten und vierten Kontaktfläche zu messen und die thermische Behandlung zu beenden, wenn die an diesen Kontaktflächen gemessene Spannung oder Stromstärke über eine festgelegte Zeitdauer (z.B. mindestens 1 Stunde oder mindestens 4 Stunden) einen konstanten Wert (z.B. eine Schwankung von maximal +/- 1%) zeigt.

Die Bestimmung des elektrischen Widerstands oder der dazu proportionalen elektrischen Größe kann beispielsweise kontinuierlich oder in definierten zeitlichen Abständen erfolgen.

Gemäß einer weiteren Ausführungsform der vorliegenden Erfindung wird die Aufgabe gelöst durch ein Verfahren zur Messung einer elektrischen Größe eines metallhaltigen Formkörpers während einer thermischen Dispersionshärtung des metallhaltigen Formkörpers, wobei

der metallhaltige Formkörper ein Metallrohr oder ein Metallstab ist, wobei das Metallrohr oder der Metallstab

eine Länge $L_{Formkörper}$ von mindestens 50 mm und einen Durchmesser $D_{Formkörper}$ im Bereich von 10 mm bis 300 mm aufweist,
an einem seiner Enden mit einer ersten Stirnfläche und an seinem gegenüberliegenden Ende mit einer zweiten Stirnfläche abschließt, die erste und zweite Stirnfläche durch eine Mantelfläche miteinander verbunden sind, die erste Stirnfläche und die zweite Stirnfläche jeweils durch eine äußere Begrenzungslinie begrenzt sind,

das Metallrohr oder der Metallstab eine erste elektrische Kontaktfläche und eine zweite elektrische Kontaktfläche aufweist und diese elektrischen Kontaktflächen mit einer Strom- oder Spannungsquelle verbunden werden, wobei

die erste elektrische Kontaktfläche vollständig innerhalb einer Fläche liegt, die aus den Schnittflächen der Mantelfläche und der ersten Stirnfläche mit einem gedachten ersten Ellipsoid besteht, wobei das gedachte erste Ellipsoid einen Mittelpunkt und Halbachsen a1, b1 und c1 aufweist, wobei der Mittelpunkt auf der äußeren Begrenzungslinie der ersten Stirnfläche des Metallrohres oder Metallstabs liegt, die Halbachse a1 parallel zu der Längsachse des Metallrohres oder Metallstabs verläuft und eine Länge $L_{a1} \leq 0{,}2 \times L_{Formkörper}$ aufweist, und die Halbachsen b1 und c1 eine Länge $L_{b1} = L_{c1} \leq 0{,}4 \times D_{Formkörper}$ aufweisen,
die zweite elektrische Kontaktfläche vollständig innerhalb einer Fläche liegt, die aus den Schnittflächen der Mantelfläche und der zweiten Stirnfläche mit einem gedachten zweiten Ellipsoid besteht, wobei das gedachte zweite Ellipsoid einen Mittelpunkt und Halbachsen a2, b2 und c2 aufweist, der Mittelpunkt des gedachten zweiten Ellipsoids der am weitesten vom Mittelpunkt des gedachten ersten Ellipsoids entfernte Punkt auf der äußeren Begrenzungslinie der zweiten Stirnfläche des Metallrohrs oder Metallstabs ist, die Halbachse a2 parallel zur Längsachse des Metallrohres oder Metallstabs verläuft und eine Länge $L_{a2} \leq 0{,}2 \times L_{Formkörper}$ aufweist, und die Halbachsen b2 und c2 eine Länge $L_{b2} = L_{c2} \leq 0{,}4 \times D_{Formkörper}$ aufweisen,

das Metallrohr oder der Metallstab eine dritte elektrische Kontaktfläche und eine vierte elektrische Kontaktfläche aufweist und diese elektrischen Kontaktflächen mit einem Spannungsmessgerät verbunden werden, sofern die erste und zweite elektrische Kontaktfläche mit einer Stromquelle verbunden werden, oder mit einem Strommessgerät verbunden werden, sofern die erste und zweite elektrische Kontaktfläche mit einer Spannungsquelle verbunden werden,
das Metallrohr oder der Metallstab, das bzw. der über seine erste und zweite elektrische Kontaktfläche mit der Strom- oder Spannungsquelle und über seine dritte und vierte elektrische Kontaktfläche mit dem Spannungs- oder Strommessgerät verbunden ist, thermisch behandelt wird, so dass sich in dem Metallrohr oder Metallstab Oxidpartikel ausbilden, und
während der thermischen Behandlung des Metallrohrs oder Metallstabs dessen elektrischer Widerstand oder eine zum elektrischen Widerstand proportionale elektrische Größe bestimmt wird.

Im Rahmen der vorliegenden Erfindung beinhaltet der Begriff "Ellipsoid" als Grenzfall auch eine Kugel.

Die Halbachsen a1, b1 und c1 des gedachten ersten Ellipsoids stehen üblicherweise senkrecht zueinander. Auch die Halbachsen a2, b2 und c2 des gedachten zweiten Ellipsoids stehen üblicherweise senkrecht zueinander.

Ist der Formkörper ein Metallrohr, so handelt es sich bei dem oben genannten Durchmesser $D_{Formkörper}$ um den Außendurchmesser des Metallrohrs. Im Falle eines Metallrohrs weisen sowohl die erste Stirnfläche wie auch die zweite Stirnfläche neben der äußeren Begrenzungslinie noch eine innere Begrenzungslinie auf.

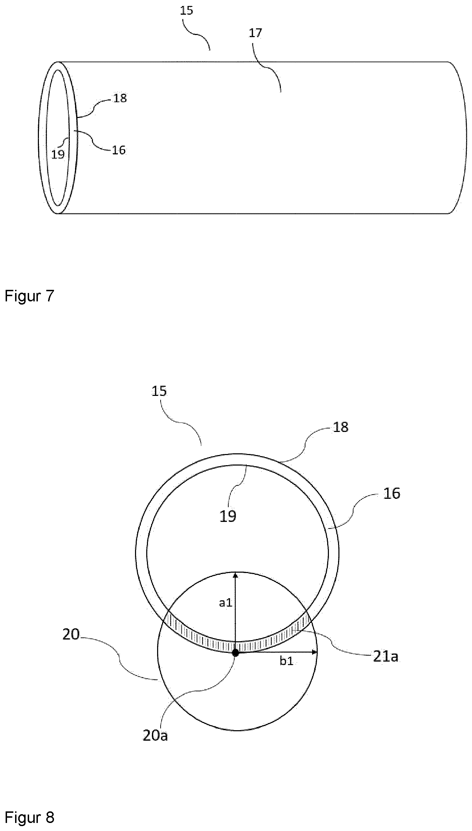

Ein beispielhaftes Metallrohr, an dem das erfindungsgemäße Messverfahren durchgeführt werden kann, wird in Figur 7 veranschaulicht.

Das in Figur 7 veranschaulichte Metallrohr 15 schließt an einem seiner Enden mit einer ersten Stirnfläche 16 ab. Die erste Stirnfläche 16 ist durch eine äußere Begrenzungslinie 18 und eine innere Begrenzungslinie 19 begrenzt. Die äußere Begrenzungslinie 18 entspricht dem Außenumfang des Rohres 15, während die innere Begrenzungslinie 19 dem Innenumfang des Rohres 15 entspricht. An dem gegenüberliegenden Ende schließt das Rohr 15 mit einer zweiten Stirnfläche (nicht gezeigt in Figur 7) ab, die ebenfalls durch eine äußere und innere Begrenzungslinie begrenzt ist. Die erste Stirnfläche 16 und die gegenüberliegende zweite Stirnfläche sind durch die Mantelfläche 17 miteinander verbunden.

Das Metallrohr oder der Metallstab weist beispielsweise eine Länge $L_{Formkörper}$ von 50 mm bis 3000 mm auf.

Das Metallrohr oder der Metallstab weist beispielsweise ein Verhältnis von $L_{Formkörper}$ zu $D_{Formkörper}$ von mindestens 2, bevorzugter mindestens 5 auf.

Das Rohr ist beispielsweise ein Rundrohr oder ein Mehrkantrohr (z.B. ein Vierkantrohr).

Die Wahl einer geeigneten Wanddicke des Rohres hängt beispielsweise von der geplanten Verwendung des Rohres ab. Beispielsweise weist das Rohr ein Verhältnis von Wanddicke zu Außendurchmesser von mindestens 0,01 auf. Das Rohr weist beispielsweise eine Wanddicke von nicht mehr als 15 mm auf.

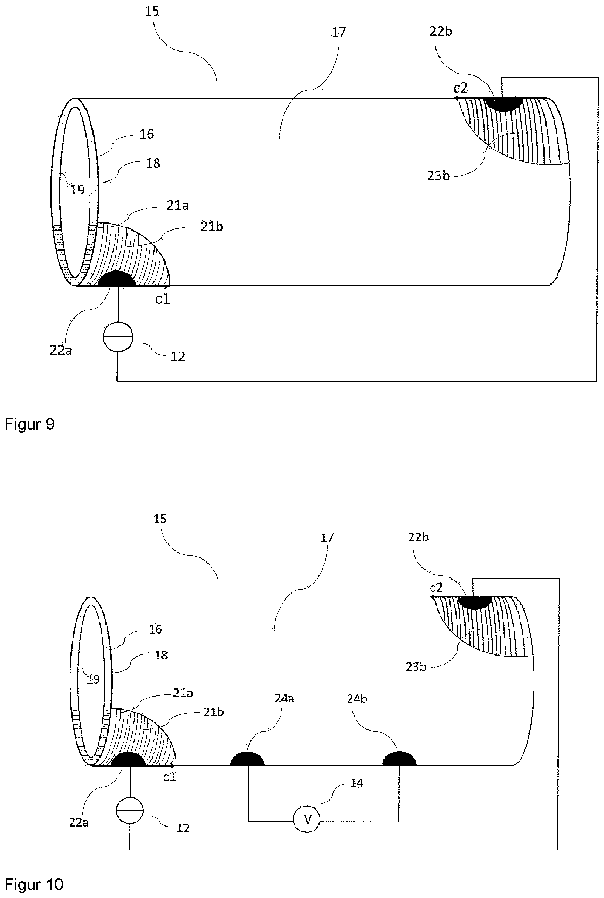

Die gedachten Ellipsoide und die Schnittflächen der Stirnfläche und Mantelfläche des Metallrohres mit diesen gedachten Ellipsoiden sind in den Figuren 8 und 9 veranschaulicht.

Figur 8 zeigt das Metallrohr 15 in Richtung seiner Längsachse. Der Mittelpunkt 20a des gedachten ersten Ellipsoids 20 liegt auf der äußeren Begrenzungslinie 18 der ersten Stirnfläche 16. Die Figur zeigt die Halbachsen a1 und b1 des Ellipsoids 20. Die erste Stirnfläche 16 schneidet das gedachte erste Ellipsoid 20 in der Schnittfläche 21a. Neben dieser Schnittfläche 21a liegt noch eine Schnittfläche der Mantelfläche des Zylinders 15 mit dem gedachten ersten Ellipsoid 20 vor (nicht gezeigt in Figur 8).

In Figur 9 schneiden die erste Stirnfläche 16 und die Mantelfläche 17 des Metallrohres 15 das gedachte erste Ellipsoid (nicht gezeigt) in den Schnittflächen 21a und 21b. Die Mantelfläche 17 schneidet das gedachte zweite Ellipsoid (nicht gezeigt) in der Schnittfläche 23b. Die Schnittfläche der zweiten Stirnfläche mit dem gedachten zweiten Ellipsoid ist in Figur 9 nicht gezeigt. Die erste elektrische Kontaktfläche 22a und die zweite elektrische Kontaktfläche 22b liegen innerhalb der Schnittflächen 21b und 23b und verbinden das Metallrohr 15 mit der Stromquelle 12.

In einer beispielhaften Ausführungsform gelten für die Längen der Halbachsen a1, b1 und c1 des gedachten ersten Ellipsoids und die Längen der Halbachsen a2, b2 und c2 des gedachten zweiten Ellipsoids folgende Beziehungen:

$$L_{a1} \leq 0,14 \times L_{Formkörper}; \ L_{b1} = L_{c1} \leq 0,25 \times D_{Formkörper};$$

$$L_{a2} \leq 0,14 \times L_{Formkörper}; \ L_{b2} = L_{c2} \leq 0,25 \times D_{Formkörper}.$$

Wie in der ersten erfindungsgemäßen Ausführungsform (d.h. Metallblech als Formkörper) wird auch in der zweiten Ausführungsform des erfindungsgemäßen Verfahrens über eine dritte elektrische Kontaktfläche und eine vierte elektrische Kontaktfläche des Metallformkörpers eine Verbindung mit einem Spannungs- oder Strommessgerät hergestellt. Sofern die erste und zweite elektrische Kontaktfläche des Metallstabs oder Metallrohrs mit einer Stromquelle verbunden werden, erfolgt eine Verbindung der dritten und vierten elektrischen Kontaktfläche des Metallstabs oder Metallrohrs mit einem Spannungsmessgerät. Sofern die erste und zweite elektrische Kontaktfläche des Metallstabs oder Metallrohrs mit einer Spannungsquelle verbunden werden, erfolgt eine Verbindung der dritten und vierten elektrischen Kontaktfläche des Metallstabs oder Metallrohrs mit einem Strommessgerät.

Für die Kontaktierung der elektrischen Kontaktflächen des Metallstabs oder Metallrohrs mit der Strom- oder Spannungsquelle und dem Spannungs- oder Strommessgerät können Kontaktelemente verwendet werden, die dem Fachmann bekannt sind. Beispielsweise erfolgt das Verbinden der elektrischen Kontaktflächen mit der Strom- oder Spannungsquelle und dem Spannungs- oder Strommessgerät über Klemmen (z.B. Kelvinklemmen oder Krokodilklemmen).

In einer bevorzugten Ausführungsform werden die erste elektrische Kontaktfläche mit einer elektrischen Leitung der Strom- oder Spannungsquelle und die dritte und vierte elektrische Kontaktfläche mit den elektrischen Leitungen des Spannungs- oder Strommessgeräts unter Einwirkung der Schwerkraft des Metallstabs oder Metallrohrs kontaktiert, beispielsweise indem der Metallstab oder das Metallrohr (z.B. mit seiner Mantelfläche oder einer seiner Stirnflächen) auf die elektrischen Leitungen gelegt oder gestellt wird. In dieser bevorzugten Ausführungsform kann die Anzahl der erforderlichen Kontaktklemmen deutlich reduziert werden.

Eine geeignete Größe der elektrischen Kontaktflächen auf dem Metallstab oder Metallrohr kann der Fachmann auf Basis seines Fachwissens und durch Routineversuche bestimmen. Beispielsweise weisen die elektrischen Kontaktflächen des Metallstabs oder Metallrohrs jeweils eine Fläche von mindestens 1 mm$^2$ auf.

In dem erfindungsgemäßen Verfahren ist wesentlich, dass die erste elektrische Kontaktfläche und die zweite elektrische Kontaktfläche des Metallstabs oder Metallrohrs (d.h. die Kontaktflächen, über die die Verbindung des Metallstabs oder Metallrohrs mit der Strom- oder Spannungsquelle erfolgt) so gewählt sind, dass sie jeweils innerhalb einer Fläche liegen, die aus den Schnittflächen der Stirnfläche und Mantelfläche mit gedachtem ersten oder zweiten Ellipsoid besteht. Dies stellt sicher, dass die erste elektrische Kontaktfläche und die zweite elektrische Kontaktfläche einen möglichst großen Abstand voneinander aufweisen.

Die Schnittflächen der Mantelfläche und ersten Stirnfläche mit dem gedachten ersten Ellipsoid sind die Flächen der Mantelfläche und ersten Stirnfläche, die innerhalb des gedachten ersten Ellipsoids liegen. Die Schnittflächen der Mantelfläche und zweiten Stirnfläche mit dem gedachten zweiten Ellipsoid sind die Flächen der Mantelfläche und zweiten Stirnfläche, die innerhalb des gedachten zweiten Ellipsoids liegen.

Die Positionen der dritten und vierten elektrischen Kontaktfläche auf dem Metallrohr oder Metallstab (d.h. die Kontaktflächen des Metallrohrs oder Metallstabs, über die die Verbindung mit dem Spannungs- oder Strommessgerät erfolgt) können frei gewählt werden. Geeignete Positionen kann der Fachmann auf Basis seines allgemeinen Fachwissens und gegebenenfalls durch Routineversuche ermitteln.

Beispielsweise weisen die dritte und vierte elektrische Kontaktfläche

von dem Mittelpunkt des gedachten ersten Ellipsoids einen größeren Abstand auf als die erste elektrische Kontaktfläche und

von dem Mittelpunkt des gedachten zweiten Ellipsoids einen größeren Abstand auf als die zweite elektrische Kontaktfläche. Der Abstand ist die kürzeste gerade Verbindungslinie zwischen der Kontaktfläche und dem Mittelpunkt des Ellipsoids.

Beispielsweise liegen die dritte und vierte elektrische Kontaktfläche außerhalb der Schnittflächen der Stirnseiten und Mantelfläche mit den gedachten Ellipsoiden.

Beispielsweise weisen die dritte und vierte elektrische Kontaktfläche einen Abstand voneinander auf, der geringer ist als der Abstand zwischen der ersten und zweiten elektrischen Kontaktfläche. Der Abstand ist die kürzeste gerade Verbindungslinie zwischen den beiden jeweiligen elektrischen Kontaktflächen.

In Figur 10 entsprechen die Schnittflächen 21a, 21b und 23 b und die elektrischen Kontaktflächen 22a und 22b (über die die Verbindung des Metallrohrs 15 mit der Stromquelle 12 erfolgt) der in Figur 9 veranschaulichten Ausführungsform. Die dritte elektrische Kontaktfläche 24a und vierte elektrische Kontaktfläche 24b, über die das Metallrohr 15 mit dem Spannungsmessgerät 14 verbunden ist, sind im Wesentlichen linear zu der ersten elektrischen Kontaktfläche 22a angeordnet. Dies kann beispielsweise dadurch realisiert werden, dass das Metallrohr 15 mit seiner Mantelfläche 17 auf die elektrischen Leitungen, die mit der Stromquelle und dem Spannungsmessgerät verbunden sind, gelegt oder gestellt wird.

Wie in der ersten erfindungsgemäßen Ausführungsform wird auch in der zweiten Ausführungsform des erfindungsgemäßen Verfahrens der metallische Formkörper, der über seine erste und zweite elektrische Kontaktfläche mit der Strom- oder Spannungsquelle und über seine dritte und vierte elektrische Kontaktflächen mit dem Spannungs- oder Strommessgerät verbunden ist, thermisch behandelt, so dass sich in dem Metallrohr Oxidpartikel ausbilden, und während der thermischen Behandlung des metallischen Formkörpers erfolgt die Bestimmung dessen elektrischen Widerstands oder einer zum elektrischen Widerstand proportionalen elektrischen Größe. Hinsichtlich weiterer Einzelheiten zu diesen Verfahrensschritten und zu geeigneten metallischen Legierungen kann somit auf die obige Beschreibung der ersten erfindungsgemäßen Ausführungsform verwiesen werden.

Außerdem betrifft die vorliegende Erfindung eine Messanordnung, enthaltend,

eine Strom- oder Spannungsquelle,

ein Spannungs- oder Strommessgerät,

einen metallhaltigen Formkörper, wobei der metallhaltige Formkörper ein Metallblech ist, das eine Vorderseite, eine der Vorderseite gegenüberliegende Rückseite und Seitenflächen, die die Vorder- und Rückseite miteinander verbinden, aufweist, wobei

die Vorderseite und die Rückseite jeweils Längskanten, deren Länge $L_{Längskante}$ mindestens 50 mm beträgt, Querkanten, deren Länge $L_{Querkante}$ mindestens 50 mm beträgt, und Ecken, in denen sich die Längs- und Querkanten treffen, aufweisen,

das Metallblech eine erste elektrische Kontaktfläche und eine zweite elektrische Kontaktfläche aufweist, die mit einer Strom- oder Spannungsquelle verbunden sind, wobei

die erste elektrische Kontaktfläche vollständig innerhalb einer Fläche liegt, die aus den Schnittflächen der Vorderseite, Rückseite und Seitenflächen mit einem gedachten ersten Zylinder besteht, wobei die Zylinderachse des gedachten ersten Zylinders senkrecht auf der Vorderseite des Metallblechs steht und durch eine der Ecken der Vorderseite läuft, der Zylindermantel des gedachten ersten Zylinders die Längskante der Vorderseite in einem Abstand a1 von der Zylinderachse und die Querkante der Vorderseite in einem Abstand b1 von der Zylinderachse schneidet, wobei

$$a1 \leq 0{,}2 \times L_{Längskante} \text{ und } b1 \leq 0{,}2 \times L_{Querkante}$$

wobei $L_{Längskante}$ die Länge der Längskante und $L_{Querkante}$ die Länge der Querkante sind, die sich in der Ecke treffen, durch die die Zylinderachse des gedachten ersten Zylinders läuft,

die zweite elektrische Kontaktfläche vollständig innerhalb einer Fläche liegt, die aus den Schnittflächen der Vorderseite, Rückseite und Seitenflächen mit einem gedachten zweiten Zylinder besteht, wobei die Zylinderachse des gedachten zweiten Zylinders senkrecht auf der Vorderseite steht und durch die Ecke der Vorderseite läuft, die zu der Ecke, durch die die Zylinderachse des gedachten ersten Zylinders läuft, den größten Abstand aufweist, der Zylindermantel des gedachten zweiten Zylinders die Längskante der Vorderseite in einem Abstand a2 von der Zylinderachse und die Querkante der Vorderseite in einem Abstand b2 von der Zylinderachse schneidet, wobei

$$a2 \leq 0{,}2 \times L_{\text{Längskante}} \text{ und } b2 \leq 0{,}2 \times L_{\text{Querkante}}$$

wobei

$L_{\text{Längskante}}$ die Länge der Längskante und $L_{\text{Querkante}}$ die Länge der Querkante sind, die sich in der Ecke treffen, durch die die Zylinderachse des gedachten zweiten Zylinders läuft,

das Metallblech eine dritte elektrische Kontaktfläche und eine vierte elektrische Kontaktfläche aufweist und diese elektrischen Kontaktflächen mit dem Spannungsmessgerät verbunden sind, sofern die erste und zweite elektrische Kontaktfläche mit der Stromquelle verbunden sind, oder mit dem Strommessgerät verbunden werden, sofern die erste und zweite elektrische Kontaktfläche mit der Spannungsquelle verbunden sind.

Mit dieser Messanordnung kann das oben beschriebene Verfahren gemäß der ersten erfindungsgemäßen Ausführungsform durchgeführt werden. Hinsichtlich weiterer Eigenschaften des Metallblechs und der elektrischen Kontaktflächen des Metallblechs kann daher auf die obige Beschreibung der ersten erfindungsgemäßen Ausführungsform verwiesen werden.

Außerdem betrifft die vorliegende Erfindung eine Messanordnung, enthaltend,

eine Strom- oder Spannungsquelle,
ein Spannungs- oder Strommessgerät,
einen metallhaltigen Formkörper, wobei der metallhaltige Formkörper ein Metallrohr oder ein Metallstab ist, wobei das Metallrohr oder der Metallstab

eine Länge $L_{\text{Formkörper}}$ von mindestens 50 mm und einen Durchmesser $D_{\text{Formkörper}}$ im Bereich von 10 mm bis 300 mm aufweist,
an einem seiner Enden mit einer ersten Stirnfläche und an seinem gegenüberliegenden Ende mit einer zweiten Stirnfläche abschließt, die Stirnflächen durch eine Mantelfläche miteinander verbunden sind und jeweils durch eine äußere Begrenzungslinie begrenzt sind,

das Metallrohr oder der Metallstab eine erste elektrische Kontaktfläche und eine zweite elektrische Kontaktfläche aufweist, die mit einer Strom- oder Spannungsquelle verbunden sind, wobei

die erste elektrische Kontaktfläche vollständig innerhalb einer Fläche liegt, die aus den Schnittflächen der Mantelfläche und der ersten Stirnfläche mit einem gedachten ersten Ellipsoid besteht, wobei das gedachte erste Ellipsoid einen Mittelpunkt und Halbachsen a1, b1 und c1 aufweist, wobei der Mittelpunkt auf der äußeren Begrenzungslinie der ersten Stirnfläche des Metallrohres oder Metallstabs liegt, die Halbachse a1 parallel zu der Längsachse des Metallrohres oder Metallstabs verläuft und eine Länge $L_{a1} \leq 0{,}2 \times L_{\text{Formkörper}}$ aufweist, und die Halbachsen b1 und c1 eine Länge $L_{b1} = L_{c1} \leq 0{,}4 \times D_{\text{Formkörper}}$ aufweisen,
die zweite elektrische Kontaktfläche vollständig innerhalb einer Fläche liegt, die aus den Schnittflächen der Mantelfläche und der zweiten Stirnfläche mit einem gedachten zweiten Ellipsoid besteht, wobei das gedachte zweite Ellipsoid einen Mittelpunkt und Halbachsen a2, b2 und c2 aufweist, der Mittelpunkt des gedachten zweiten Ellipsoids der am weitesten vom Mittelpunkt des gedachten ersten Ellipsoids entfernte Punkt auf der äußeren Begrenzungslinie der zweiten Stirnfläche des Metallrohrs oder Metallstabs ist, die Halbachse a2 parallel zur Längsachse des Metallrohres oder Metallstabs verläuft und eine Länge $L_{a2} \leq 0{,}2 \times L_{\text{Formkörper}}$ aufweist, und die Halbachsen b2 und c2 eine Länge $L_{b2} = L_{c2} \leq 0{,}4 \times D_{\text{Formkörper}}$ aufweisen,

das Metallrohr oder der Metallstab eine dritte elektrische Kontaktfläche und eine vierte elektrische Kontaktfläche aufweist und diese elektrischen Kontaktflächen mit einem Spannungsmessgerät verbunden sind, sofern die erste und zweite elektrische Kontaktfläche mit einer Stromquelle verbunden sind, oder mit einem Strommessgerät verbunden sind, sofern die erste und zweite elektrische Kontaktfläche mit einer Spannungsquelle verbunden sind.

Mit dieser Messanordnung kann das oben beschriebene Verfahren gemäß der zweiten erfindungsgemäßen Ausführungsform durchgeführt werden. Hinsichtlich weiterer Eigenschaften des Metallrohrs oder Metallstabs und der elektrischen Kontaktflächen des Metallrohrs oder Metallstabs kann daher auf die obige Beschreibung der zweiten erfindungsgemäßen Ausführungsform verwiesen werden.

Die vorliegende Erfindung betrifft außerdem die Verwendung der oben beschriebenen Messanordnungen zur Bestimmung der Zeitdauer einer thermischen Dispersionshärtung des Metallblechs, Metallstabs oder Metallrohres.

Die vorliegende Erfindung wird anhand der nachfolgenden Beispiele eingehender erläutert.

**Beispiele**

Es wurden zwei Ausgangsmetallbleche hergestellt, die übereinstimmende Abmessungen und eine übereinstimmende chemische Zusammensetzung aufwiesen.

$L_{Längskante}$ (d.h. Länge der Längskante der Bleche): 150 mm
$L_{Querkante}$ (d.h. Länge der Querkante der Bleche): 120 mm

Für die Herstellung der beiden Ausgangsbleche wurde eine Platin-Legierung, wie sie in Beispiel 3 der EP 3971311 B1 beschrieben ist, verwendet.

Jedes der in ihrer chemischen Zusammensetzung übereinstimmenden Ausgangsbleche wurde anschließend unter identischen Bedingungen in einer oxidativen Atmosphäre einer thermischen Dispersionshärtung unterzogen. Die Dispersionshärtung erfolgte jeweils in einer LuftAtmosphäre bei einer Temperatur von 1000°C.

Mit der Dispersionshärtung ist beabsichtigt, die Nichtedelmetalle der Legierung möglichst vollständig zu oxidieren (unter Bildung von Oxidpartikeln, die in der Edelmetallmatrix dispergiert sind).

Für beide Metallbleche wurde während der Dispersionshärtung der elektrische Widerstand als Funktion der Zeit über eine Vierleitermessung bestimmt. Wie nachfolgend eingehender beschrieben, wurden jedoch unterschiedliche Messanordnungen für die Vierleitermessung verwendet.

Die Messanordnungen des Vergleichsbeispiels und des erfindungsgemäßen Beispiels unterschieden sich in der relativen Anordnung der ersten und zweiten elektrischen Kontaktfläche zueinander.

In der Messanordnung des Vergleichsbeispiels lag die erste elektrische Kontaktfläche auf einer der Seitenflächen nahe einer ersten Ecke des Blechs und die zweite elektrische Kontaktfläche lag auf derselben Seitenfläche nahe der zur ersten Ecke benachbarten Ecke.

Abstand der ersten elektrischen Kontaktfläche von der ersten Ecke: 15 mm Abstand der zweiten elektrischen Kontaktfläche von der zur ersten Ecke benachbarten Ecke: 15 mm

In der Messanordnung des erfindungsgemäßen Beispiels wurde die Position der zweiten elektrischen Kontaktfläche so verändert, dass sie sich auf der gegenüberliegenden Seitenfläche in der Nähe der Ecke befand, die der ersten Ecke gegenüberlag.

Abstand der zweiten elektrischen Kontaktfläche von der gegenüberliegenden Ecke: 15 mm

Figur 6 zeigt eine schematische Darstellung der im erfindungsgemäßen Beispiel verwendeten Messanordung.

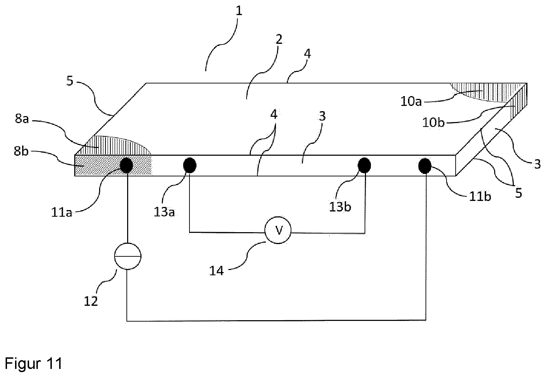

Eine schematische Darstellung der Messanordnung des Vergleichsbeispiels zeigt die Figur 11. In Übereinstimmung mit Figur 6 zeigt die Figur 11 ein Metallblech 1 mit einer Vorderseite 2, Seitenflächen 3, Längskanten 4, Querkanten 5 und Schnittflächen 8a, 8b, 10a, 10b der Vorderseite 2 und Seitenflächen 3 mit den gedachten Zylindern. Die Spannungsquelle 14 ist über die dritte und vierte elektrische Kontaktfläche 13a und 13b mit dem Metallblech 1 verbunden. Die Stromquelle 12 ist über die erste und zweite elektrische Kontaktfläche 11a und 11b mit dem Metallblech verbunden. Im Unterschied zu dem erfindungsgemäßen Beispiel liegt in dem Vergleichsbeispiel die zweite elektrische Kontaktfläche 11b nicht auf einer der Schnittflächen der Vorderseite, Rückseite oder Seitenflächen des Metallblechs mit dem gedachten zweiten Zylinder vor.

In beiden Fällen wurde die thermische Behandlung beendet, wenn der elektrische Widerstand für einen Zeitraum von 2 Stunden einen konstanten Wert aufwies.

Mit der erfindungsgemäßen Messanordnung ergab sich ein konstanter Widerstandswert nach 144 Stunden.

Mit der Messanordung des Vergleichsbeispiels ergab sich ein konstanter Widerstandswert nach 60 Stunden.

Für beide Bleche wurde nach Beendigung der Dispersionshärtung deren Sauerstoffgehalt über quantitative IR-Spektroskopie mit einem Gerät der Firma LECO (ONH836) bestimmt. Weitere Einzelheiten zu dieser Methode finden sich in EP 3 971 311 B1. Bei Kenntnis der chemischen Zusammensetzung der Legierung lässt sich aus dem gemessenen Sauerstoff der Oxidationsgrad errechnen. Der Oxidationsgrad gibt an, zu welchem Anteil die Nichtedelmetalle während der Dispersionshärtung in die entsprechenden Oxide überführt wurden. Erwünscht wäre ein Wert, der möglichst nahe bei 100% liegt.

Die Ergebnisse sind in der folgenden Tabelle zusammengefasst:

| | Zeitdauer [h] bis zur Erreichung eines für mindestens 2 Stunden konstanten Widerstandwerts | Oxidationsgrad [%] |
|---|---|---|
| Messanordung des Vergleichsbeispiels | 60 | 50 |
| Messanordung des erfindungsgemäßen Beispiels | 144 | 100 |

Die Daten zeigen, dass sich mit der erfindungsgemäßen Messanordnung die für die Dispersionshärtung erforderliche Zeitdauer wesentlich verlässlicher bestimmen lässt.

Liste der Bezugszeichen:

| | |
|---|---|
| 1 | Metallblech |
| 2 | Vorderseite des Metallblechs |
| 3 | Seitenflächen des Metallblechs |
| 4 | Längskanten der Vorder- und Rückseite des Metallblechs |
| 5 | Querkanten der Vorder- und Rückseite des Metallblechs |
| 6 | Ecken des Metallblechs |
| 7 | gedachter erster Zylinder |
| 7a | Zylinderachse des gedachten ersten Zylinders |
| 7b | Zylindermantel des gedachten ersten Zylinders |
| 8a, 8b | Schnittflächen der Vorderseite und Seitenflächen des Metallblechs mit dem gedachten ersten Zylinder |
| 9 | gedachter zweiter Zylinder |
| 9a | Zylinderachse des gedachten zweiten Zylinders |
| 9b | Zylindermantel des gedachten zweiten Zylinders |
| 10a, 10b | Schnittflächen der Vorderseite und Seitenflächen des Metallblechs mit dem gedachten zweiten Zylinder |
| 11a | erste elektrische Kontaktfläche des Metallblechs |
| 11b | zweite elektrische Kontaktfläche des Metallblechs |
| 12 | Strom- oder Spannungsquelle |
| 13a, 13b | dritte und vierte elektrische Kontaktfläche des Metallblechs |
| 14 | Spannungs- oder Strommessgerät |
| 15 | Metallrohr |
| 16 | erste Stirnfläche des Metallrohrs |
| 17 | Mantelfläche des Metallrohrs |
| 18 | äußere Begrenzungslinie der ersten Stirnfläche |
| 19 | innere Begrenzungslinie der ersten Stirnfläche |
| 20 | gedachtes erstes Ellipsoid |
| 20a | Mittelpunkt des gedachten ersten Ellipsoids |
| 21a, 21b | Schnittflächen der ersten Stirnfläche und der Mantelfläche mit dem gedachten ersten Ellipsoid |
| 22a | erste elektrische Kontaktfläche des Metallrohrs |
| 22b | zweite elektrische Kontaktfläche des Metallrohrs |
| 23b | Schnittfläche der Mantelfläche mit dem gedachten zweiten Ellipsoid |
| 24a, 24b | dritte und vierte elektrische Kontaktfläche des Metallrohrs |

**Patentansprüche**

1. Ein Verfahren zur Messung einer elektrischen Größe eines metallhaltigen Formkörpers während einer thermischen Dispersionshärtung des metallhaltigen Formkörpers, wobei

der metallhaltige Formkörper ein Metallblech (1) ist, das eine Vorderseite (2), eine der Vorderseite (2) gegenüberliegende Rückseite und Seitenflächen (3), die die Vorderseite (2) und Rückseite miteinander verbinden, aufweist, wobei
die Vorderseite (2) und die Rückseite jeweils Längskanten (4), deren Länge $L_{Längskante}$ mindestens 50 mm beträgt, Querkanten (5), deren Länge $L_{Querkante}$ mindestens 50 mm beträgt, und Ecken (6), in denen sich die Längskanten (4) und Querkanten (5) treffen, aufweisen,

das Metallblech (1) eine erste elektrische Kontaktfläche (11a) und eine zweite elektrische Kontaktfläche (11b) aufweist, die mit einer Strom- oder Spannungsquelle (12) verbunden werden, wobei

die erste elektrische Kontaktfläche (11a) vollständig innerhalb einer Fläche liegt, die aus den Schnittflächen (8a, 8b) der Vorderseite, Rückseite und Seitenflächen mit einem gedachten ersten Zylinder (7) besteht, wobei die Zylinderachse (7a) des gedachten ersten Zylinders (7) senkrecht auf der Vorderseite (2) des Metallblechs (1) steht und durch eine der Ecken (6) der Vorderseite (2) läuft, der Zylindermantel (7b) des gedachten ersten Zylinders (7) die Längskante (4) der Vorderseite (2) in einem Abstand a1 von der Zylinderachse (7a) und die Querkante (5) der Vorderseite (2) in einem Abstand b1 von der Zylinderachse (7a) schneidet, wobei

$$a1 \leq 0{,}2 \times L_{\text{Längskante}} \text{ und } b1 \leq 0{,}2 \times L_{\text{Querkante}}$$

wobei
$L_{\text{Längskante}}$ die Länge der Längskante (4) und $L_{\text{Querkante}}$ die Länge der Querkante (5) sind, die sich in der Ecke (6) treffen, durch die die Zylinderachse (7a) des gedachten ersten Zylinders (7) läuft,
die zweite elektrische Kontaktfläche (11b) vollständig innerhalb einer Fläche liegt, die aus den Schnittflächen (10a, 10b) der Vorderseite, Rückseite und Seitenflächen mit einem gedachten zweiten Zylinder (9) besteht, wobei die Zylinderachse (9a) des gedachten zweiten Zylinders (9) senkrecht auf der Vorderseite (2) steht und durch die Ecke (6) der Vorderseite (2) läuft, die zu der Ecke (6), durch die die Zylinderachse (7a) des gedachten ersten Zylinders (7) läuft, den größten Abstand aufweist, der Zylindermantel (9b) des gedachten zweiten Zylinders (9) die Längskante (4) der Vorderseite (2) in einem Abstand a2 von der Zylinderachse (9a) und die Querkante (5) der Vorderseite (2) in einem Abstand b2 von der Zylinderachse (9a) schneidet, wobei

$$a2 \leq 0{,}2 \times L_{\text{Längskante}} \text{ und } b2 \leq 0{,}2 \times L_{\text{Querkante}}$$

wobei
$L_{\text{Längskante}}$ die Länge der Längskante (4) und $L_{\text{Querkante}}$ die Länge der Querkante (5) sind, die sich in der Ecke (6) treffen, durch die die Zylinderachse (9a) des gedachten zweiten Zylinders (9) läuft,

das Metallblech (1) eine dritte elektrische Kontaktfläche (13a) und eine vierte elektrische Kontaktfläche (13b) aufweist, die mit einem Spannungsmessgerät (14) verbunden werden, sofern die erste und zweite elektrische Kontaktfläche (11a, 11b) mit einer Stromquelle (12) verbunden werden, oder die mit einem Strommessgerät (14) verbunden werden, sofern die erste und zweite elektrische Kontaktfläche (11a, 11b) mit einer Spannungsquelle (12) verbunden werden,
das Metallblech (1), das über seine erste und zweite elektrische Kontaktfläche (11a, 11b) mit der Strom- oder Spannungsquelle (12) und über seine dritte und vierte elektrische Kontaktfläche (13a, 13b) mit dem Spannungs- oder Strommessgerät (14) verbunden ist, thermisch behandelt wird, so dass sich in dem Metallblech (1) Oxidpartikel ausbilden und
während der thermischen Behandlung der elektrische Widerstand oder eine zum elektrischen Widerstand proportionale elektrische Größe des Metallblechs (1) bestimmt wird.

2. Das Verfahren nach Anspruch 1, wobei die erste und zweite elektrische Kontaktfläche (11a, 11b) auf gegenüberliegenden Seitenflächen (3) des Metallblechs (1) vorliegen.

3. Das Verfahren nach Anspruch 1 oder 2, wobei die dritte und vierte elektrische Kontaktfläche (13a, 13b) einen Abstand voneinander aufweisen, der geringer ist als der Abstand zwischen der ersten und zweiten elektrischen Kontaktfläche (11a, 11b); und/oder wobei die dritte und vierte elektrische Kontaktfläche (13a, 13b) außerhalb der Schnittflächen (8a, 8b, 10a, 10b) der Vorderseite, Rückseite und Seitenflächen des Metallblechs mit dem gedachten ersten und zweiten Zylinder (7, 9) liegen.

4. Das Verfahren nach einem der vorstehenden Ansprüche, wobei die dritte und vierte elektrische Kontaktfläche (13a, 13b) und die erste elektrische Kontaktfläche (11a) auf derselben Seitenfläche (3) des Metallblechs (1) vorliegen und die zweite elektrische Kontaktfläche (11b) auf einer anderen Seitenfläche (3), bevorzugt der gegenüberliegenden Seitenfläche (3) des Metallblechs (1) vorliegt.

5. Das Verfahren nach einem der vorstehenden Ansprüche, wobei die erste oder zweite elektrische Kontaktfläche (11a, 11b) mit einer elektrischen Leitung der Strom- oder Spannungsquelle (12) und die dritte und vierte elektrische Kontaktfläche (13a, 13b) mit den elektrischen Leitungen des Spannungs- oder Strommessgeräts (14) unter Einwirkung der Schwerkraft des Metallblechs (1) kontaktiert werden, indem das Metallblech (1) über seine Vorderseite (2), Rückseite oder eine seiner Seitenflächen (3) auf die elektrischen Leitungen gelegt oder gestellt wird.

6. Ein Verfahren zur Messung einer elektrischen Größe eines metallhaltigen Formkörpers während einer thermischen Dispersionshärtung des metallhaltigen Formkörpers, wobei

der metallhaltige Formkörper ein Metallrohr (15) oder ein Metallstab ist, wobei das Metallrohr (15) oder der Metallstab

eine Länge $L_{Formkörper}$ von mindestens 50 mm und einen Durchmesser $D_{Formkörper}$ im Bereich von 10 mm bis 300 mm aufweist,

an einem seiner Enden mit einer ersten Stirnfläche (16) und an seinem gegenüberliegenden Ende mit einer zweiten Stirnfläche abschließt, die erste und zweite Stirnfläche (16) durch eine Mantelfläche (17) miteinander verbunden sind, die erste Stirnfläche (16) und die zweite Stirnfläche jeweils durch eine äußere Begrenzungslinie (18) begrenzt sind,

das Metallrohr (15) oder der Metallstab eine erste elektrische Kontaktfläche (22a) und eine zweite elektrische Kontaktfläche (22b) aufweist und diese elektrischen Kontaktflächen mit einer Strom- oder Spannungsquelle (12) verbunden werden, wobei

die erste elektrische Kontaktfläche (22a) vollständig innerhalb einer Fläche liegt, die aus den Schnittflächen (21a, 21b) der Mantelfläche (17) und der ersten Stirnfläche (16) mit einem gedachten ersten Ellipsoid (20) besteht, wobei das gedachte erste Ellipsoid (20) einen Mittelpunkt (20a) und Halbachsen a1, b1 und c1 aufweist, wobei der Mittelpunkt (20a) auf der äußeren Begrenzungslinie (18) der ersten Stirnfläche (16) des Metallrohres (15) oder Metallstabs liegt, die Halbachse a1 parallel zu der Längsachse des Metallrohres (15) oder Metallstabs verläuft und eine Länge $L_{a1} \leq 0{,}2 \times L_{Formkörper}$ aufweist, und die Halbachsen b1 und c1 eine Länge $L_{b1} = L_{c1} \leq 0{,}4 \times D_{Formkörper}$ aufweisen, die zweite elektrische Kontaktfläche (22b) vollständig innerhalb einer Fläche liegt, die aus den Schnittflächen (23b) der Mantelfläche (17) und der zweiten Stirnfläche mit einem gedachten zweiten Ellipsoid besteht, wobei das gedachte zweite Ellipsoid einen Mittelpunkt und Halbachsen a2, b2 und c2 aufweist, der Mittelpunkt des gedachten zweiten Ellipsoids der am weitesten vom Mittelpunkt (20a) des gedachten ersten Ellipsoids (20) entfernte Punkt auf der äußeren Begrenzungslinie der zweiten Stirnfläche des Metallrohrs (15) oder Metallstabs ist, die Halbachse a2 parallel zur Längsachse des Metallrohres (15) oder Metallstabs verläuft und eine Länge $L_{a2} \leq 0{,}2 \times L_{Formkörper}$ aufweist, und die Halbachsen b2 und c2 eine Länge $L_{b2} = L_{c2} \leq 0{,}4 \times D_{Formkörper}$ aufweisen,

das Metallrohr (15) oder der Metallstab eine dritte elektrische Kontaktfläche (24a) und eine vierte elektrische Kontaktfläche (24b) aufweist und diese elektrischen Kontaktflächen (24a, 24b) mit einem Spannungsmessgerät (14) verbunden werden, sofern die erste und zweite elektrische Kontaktfläche (22a, 22b) mit einer Stromquelle (12) verbunden werden, oder mit einem Strommessgerät (14) verbunden werden, sofern die erste und zweite elektrische Kontaktfläche (22a, 22b) mit einer Spannungsquelle (12) verbunden werden, das Metallrohr (15) oder der Metallstab, das bzw. der über seine erste und zweite elektrische Kontaktfläche (22a, 22b) mit der Strom- oder Spannungsquelle (12) und über seine dritte und vierte elektrische Kontaktfläche (24a, 24b) mit dem Spannungs- oder Strommessgerät (14) verbunden ist, thermisch behandelt wird, so dass sich in dem Metallrohr (15) oder Metallstab Oxidpartikel ausbilden, und während der thermischen Behandlung des Metallrohrs (15) oder Metallstabs dessen elektrischer Widerstand oder eine zum elektrischen Widerstand proportionale elektrische Größe bestimmt wird.

7. Das Verfahren nach Anspruch 6, wobei der Durchmesser $D_{Formkörper}$ des Metallrohrs (15) sein Außendurchmesser ist und die erste und zweite Stirnfläche (16) des Metallrohrs neben der äußeren Begrenzungslinie (18) jeweils noch eine innere Begrenzungslinie (19) aufweist.

8. Das Verfahren nach Anspruch 6 oder 7, wobei die dritte und vierte elektrische Kontaktfläche (24a, 24b) einen Abstand voneinander aufweisen, der geringer ist als der Abstand zwischen der ersten und zweiten elektrischen Kontaktfläche (22a, 22b); und/oder wobei die dritte und vierte elektrische Kontaktfläche (24a, 24b) außerhalb der Schnittflächen (21a, 21b, 23b) der Stirnflächen (16) und Mantelfläche (17) mit dem gedachten ersten und zweiten Ellipsoid (20) liegen.

9. Das Verfahren nach einem der Ansprüche 6-8, wobei die erste oder zweite elektrische Kontaktfläche (22a, 22b) mit einer elektrischen Leitung der Strom- oder Spannungsquelle (12) und die dritte und vierte elektrische Kontaktfläche (24a, 24b) mit den elektrischen Leitungen des Spannungs- oder Strommessgeräts (14) unter Einwirkung der Schwerkraft des Metallstabs oder Metallrohrs (15) kontaktiert wird, indem der Metallstab oder das Metallrohr (15) mit seiner Mantelfläche (17) oder einer seiner Stirnflächen (16) auf die elektrischen Leitungen gelegt oder gestellt wird.

10. Das Verfahren nach einem der vorstehenden Ansprüche, wobei das Metall, aus dem das Metallblech (1), der Metallstab oder das Metallrohr (15) gefertigt ist, eine Edelmetalllegierung ist, die ein oder mehrere Nichtedelmetalle enthält.

11. Das Verfahren nach Anspruch 10, wobei das Nichtedelmetall ausgewählt ist aus Zirkonium, Cer, Scandium und Yttrium und in der Edelmetalllegierung in einer Gesamtkonzentration von nicht mehr als 1 Gew% vorliegt und der Rest ein oder mehrere Edelmetalle, ausgewählt aus Ruthenium, Rhodium, Gold, Palladium, Platin, Iridium und Osmium, und unvermeidliche Verunreinigungen sind.

12. Das Verfahren nach einem der vorstehenden Ansprüche, wobei die thermische Behandlung des Metallblechs (1), Metallrohrs (15) oder Metallstabs bei einer Temperatur von mindestens 750°C erfolgt.

13. Das Verfahren nach einem der vorstehenden Ansprüche, wobei die thermische Behandlung des Metallblechs (1), Metallrohrs (15) oder Metallstabs beendet wird, wenn der elektrische Widerstand oder die dazu proportionale elektrische Größe des Metallblechs (1), Metallrohrs (15) oder Metallstabs über eine festgelegte Mindestzeitdauer einen konstanten Wert aufweist.

14. Eine Messanordnung, enthaltend,

eine Strom- oder Spannungsquelle (12),
ein Spannungs- oder Strommessgerät (14),
einen metallhaltigen Formkörper, wobei der metallhaltige Formkörper ein Metallblech (1) ist, das eine Vorderseite (2), eine der Vorderseite gegenüberliegende Rückseite und Seitenflächen (3), die die Vorderseite (2) und Rückseite miteinander verbinden, aufweist, wobei
die Vorderseite (2) und die Rückseite jeweils Längskanten (4), deren Länge $L_{Längskante}$ mindestens 50 mm beträgt, Querkanten (5), deren Länge $L_{Querkante}$ mindestens 50 mm beträgt, und Ecken (6), in denen sich die Längskanten (4) und Querkanten (5) treffen, aufweisen,
das Metallblech (1) eine erste elektrische Kontaktfläche (11a) und eine zweite elektrische Kontaktfläche (11b) aufweist, die mit einer Strom- oder Spannungsquelle (12) verbunden sind, wobei

die erste elektrische Kontaktfläche (11a) vollständig innerhalb einer Fläche liegt, die aus den Schnittflächen (8a, 8b) der Vorderseite (2), Rückseite und Seitenflächen (3) mit einem gedachten ersten Zylinder (7) besteht, wobei die Zylinderachse (7a) des gedachten ersten Zylinders (7) senkrecht auf der Vorderseite (2) des Metallblechs (1) steht und durch eine der Ecken (6) der Vorderseite (2) läuft, der Zylindermantel (7b) des gedachten ersten Zylinders (7) die Längskante (4) der Vorderseite (2) in einem Abstand a1 von der Zylinderachse (7a) und die Querkante (5) der Vorderseite (2) in einem Abstand b1 von der Zylinderachse (7a) schneidet, wobei

$$a1 \leq 0,2 \times L_{Längskante} \text{ und } b1 \leq 0,2 \times L_{Querkante}$$

wobei
$L_{Längskante}$ die Länge der Längskante (4) und $L_{Querkante}$ die Länge der Querkante (5) sind, die sich in der Ecke (6) treffen, durch die die Zylinderachse (7a) des gedachten ersten Zylinders (7) läuft,
die zweite elektrische Kontaktfläche (11b) vollständig innerhalb einer Fläche liegt, die aus den Schnittflächen (10a, 10b) der Vorderseite (2), Rückseite und Seitenflächen (3) mit einem gedachten zweiten Zylinder (9) besteht, wobei die Zylinderachse (9a) des gedachten zweiten Zylinders (9) senkrecht auf der Vorderseite (2) steht und durch die Ecke (6) der Vorderseite (2) läuft, die zu der Ecke (6), durch die die Zylinderachse (7a) des gedachten ersten Zylinders (7) läuft, den größten Abstand aufweist, der Zylindermantel (9b) des gedachten zweiten Zylinders (9) die Längskante (4) der Vorderseite (2) in einem Abstand a2 von der Zylinderachse (9a) und die Querkante (5) der Vorderseite (2) in einem Abstand b2 von der Zylinderachse (9a) schneidet, wobei

$$a2 \leq 0{,}2 \times L_{\text{Längskante}} \text{ und } b2 \leq 0{,}2 \times L_{\text{Querkante}}$$

wobei

$L_{\text{Längskante}}$ die Länge der Längskante (4) und $L_{\text{Querkante}}$ die Länge der Querkante (5) sind, die sich in der Ecke (6) treffen, durch die die Zylinderachse (9a) des gedachten zweiten Zylinders (9) läuft,

das Metallblech (1) eine dritte elektrische Kontaktfläche (13a) und eine vierte elektrische Kontaktfläche (13b) aufweist und diese elektrischen Kontaktflächen (13a, 13b) mit dem Spannungsmessgerät (14) verbunden sind, sofern die erste und zweite elektrische Kontaktfläche (11a, 11b) mit der Stromquelle (12) verbunden sind, oder mit dem Strommessgerät (14) verbunden sind, sofern die erste und zweite elektrische Kontaktfläche (11a, 11b) mit der Spannungsquelle (12) verbunden sind.

15. Eine Messanordnung, enthaltend,

eine Strom- oder Spannungsquelle (12),
ein Spannungs- oder Strommessgerät (14),
einen metallhaltigen Formkörper, wobei der der metallhaltige Formkörper ein Metallrohr (15) oder ein Metallstab ist, wobei das Metallrohr (15) oder der Metallstab

eine Länge $L_{\text{Formkörper}}$ von mindestens 50 mm und einen Durchmesser $D_{\text{Formkörper}}$ im Bereich von 10 mm bis 300 mm aufweist,
an einem seiner Enden mit einer ersten Stirnfläche (16) und an seinem gegenüberliegenden Ende mit einer zweiten Stirnfläche abschließt, die Stirnflächen (16) durch eine Mantelfläche (17) miteinander verbunden sind und jeweils durch eine äußere Begrenzungslinie (18) begrenzt sind,

das Metallrohr (15) oder der Metallstab eine erste elektrische Kontaktfläche (22a) und eine zweite elektrische Kontaktfläche (22b) aufweist, die mit der Strom- oder Spannungsquelle (12) verbunden sind, wobei

die erste elektrische Kontaktfläche (22a) vollständig innerhalb einer Fläche liegt, die aus den Schnittflächen (21a, 21b) der Mantelfläche (17) und der ersten Stirnfläche (16) mit einem gedachten ersten Ellipsoid (20) besteht, wobei das gedachte erste Ellipsoid (20) einen Mittelpunkt (20a) und Halbachsen a1, b1 und c1 aufweist, wobei der Mittelpunkt (20a) auf der äußeren Begrenzungslinie (18) der ersten Stirnfläche (16) des Metallrohres (15) oder Metallstabs liegt, die Halbachse a1 parallel zu der Längsachse des Metallrohres (15) oder Metallstabs verläuft und eine Länge $L_{a1} \leq 0{,}2 \times L_{\text{Formkörper}}$ aufweist, und die Halbachsen b1 und c1 eine Länge $L_{b1} = L_{c1} \leq 0{,}4 \times D_{\text{Formkörper}}$ aufweisen,
die zweite elektrische Kontaktfläche (22b) vollständig innerhalb einer Fläche liegt, die aus den Schnittflächen (23b) der Mantelfläche (17) und der zweiten Stirnfläche mit einem gedachten zweiten Ellipsoid besteht, wobei das gedachte zweite Ellipsoid einen Mittelpunkt und Halbachsen a2, b2 und c2 aufweist, der Mittelpunkt des gedachten zweiten Ellipsoids der am weitesten vom Mittelpunkt (20a) des gedachten ersten Ellipsoids (20) entfernte Punkt auf der äußeren Begrenzungslinie der zweiten Stirnfläche des Metallrohrs (15) oder Metallstabs ist, die Halbachse a2 parallel zur Längsachse des Metallrohres (15) oder Metallstabs verläuft und eine Länge $L_{a2} \leq 0{,}2 \times L_{\text{Formkörper}}$ aufweist, und die Halbachsen b2 und c2 eine Länge $L_{b2} = L_{c2} \leq 0{,}4 \times D_{\text{Formkörper}}$ aufweisen,

das Metallrohr (15) oder der Metallstab eine dritte elektrische Kontaktfläche (24a) und eine vierte elektrische Kontaktfläche (24b) aufweist und diese elektrischen Kontaktflächen (24a, 24b) mit einem Spannungsmessgerät (14) verbunden sind, sofern die erste und zweite elektrische Kontaktfläche (22a, 22b) mit einer Stromquelle (12) verbunden sind, oder mit einem Strommessgerät (14) verbunden sind, sofern die erste und zweite elektrische Kontaktfläche (22a, 22b) mit einer Spannungsquelle (12) verbunden sind.

16. Verwendung der Messanordnung nach Anspruch 14 oder 15 zur Bestimmung der Zeitdauer einer thermischen Dispersionshärtung des Metallblechs (1), Metallstabs oder Metallrohrs (15).

Figur 1

Figur 2

Figur 3

Figur 4

Figur 5

Figur 6

15

17

18

19    16

Figur 7

15

18

19    16

20

a1

b1    21a

20a

Figur 8

Figur 9

Figur 10

Figur 11

**EP 4 492 048 A1**

Europäisches Patentamt
European Patent Office
Office européen des brevets

**EUROPÄISCHER RECHERCHENBERICHT**

Nummer der Anmeldung

**EP 23 18 5034**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | COSOVIC VLADAN ET AL: "Improving dispersion of SnO2nanoparticles in Ag-SnO2electrical contact materials using template me", JOURNAL OF ALLOYS AND COMPOUNDS, ELSEVIER SEQUOIA, LAUSANNE, CH, Bd. 567, 21. März 2013 (2013-03-21), Seiten 33-39, XP028586591, ISSN: 0925-8388, DOI: 10.1016/J.JALLCOM.2013.03.094 * das ganze Dokument * ----- | 1-16 | INV. G01N27/04 G01N33/208 |

RECHERCHIERTE SACHGEBIETE (IPC)

G01N

Der vorliegende Recherchenbericht wurde für alle Patentansprüche erstellt

| Recherchenort | Abschlußdatum der Recherche | Prüfer |
|---|---|---|
| **München** | **7. November 2023** | **Klein, Marc-Oliver** |

EPO FORM 1503 03.82 (P04C03)

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 3971311 B1
- GB 1280815 A
- GB 1340076 A
- GB 2082205 A
- EP 0683240 A2
- EP 0870844 A1
- EP 0947595 A2
- EP 1188844 A1
- EP 1295953 A1
- EP 1964938 A1
- US 2636819 A
- US 4507156 A
- DE 2355122 A1
- WO 8101013 A1
- WO 2015082630 A1

**In der Beschreibung aufgeführte Nicht-Patentliteratur**

- **G. GOTTSTEIN**. Materialwissenschaft und Werkstofftechnik-Physikalische Grundlagen. SpringerVieweg, 2014, 276
- Metallurgy for the Non-Metallurgist. ASM International, 2011, 69-70
- **W.D. CALLISTER** ; **D.G. RETHWISCH**. Materials Science and Engineering - An Introduction. Wiley, 2018, 571
- **M. BRUNCKO et al.** In-situ monitoring of internal oxidation ofdilute alloys. *Corrosion Science*, 2007, vol. 49, 1228-1244